# EUROPEAN PATENT SPECIFICATION

(11) **EP 1 727 902 B1**
(45) Date of publication and mention of the grant of the patent: **03.09.2008**
(21) Application number: 05758606.7
(22) Date of filing: 18.02.2005
(51) Int. Cl.: C12N 15/52, C12N 15/70, C12N 9/88, C12P 7/42, C12P 13/02

(54) **EXPRESSION OF NITRILE HYDRATASES IN A TWO-VECTOR EXPRESSION SYSTEM**
EXPRESSION VON NITRILHYDRATASEN IN EINEM ZWEIVEKTOREXPRESSIONSSYSTEM
EXPRESSION DE NITRILE HYDRATASES DANS UN SYSTEME D'EXPRESSION A DEUX VECTEURS

(30) Priority: 20.03.2004 DE 102004013843
(43) Date of publication of application: 06.12.2006
(73) Proprietor: B.R.A.I.N. Biotechnology Research And Information Network AG, 64673 Zwingenberg (DE)
(72) Inventor: VERSECK, Stefan, 63456 Hanau (DE); OSSWALD, Steffen, 63517 Rodenbach (DE); PHONG, Wai-Yee, SG-380056 (SG); LIEBETON, Klaus, 64673 Zwingenberg (DE); ECK, Jürgen, 64625 Bensheim (DE)
(74) Representative: Vossius & Partner
(86) International application number: PCT/EP2005/001688
(87) International publication number: WO 2005/090577

(56) References cited:
- WO-A-95/12662
- WO-A-20/04067738
- NOJIRI M ET AL: "FUNCTIONAL EXPRESSION OF NITRILE HYDRATASE IN ESCHERICHIA COLI: REGUIREMENT OF A NITRILE HYDRATASE ACTIVATOR AND POST-TRANSLATIONAL MODIFICATION OF A LIGAND CYSTEINE" JOURNAL OF BIOCHEMISTRY, JAPANESE BIOCHEMICAL SOCIETY, TOKYO, JP, vol. 125, no. 4, April 1999 (1999-04), pages 696-704, XP009049760 ISSN: 0021-924X
- SKOWRONEK KRZYSZTOF ET AL: "A two-plasmid system for independent genetic manipulation of subunits of homodimeric proteins and selective isolation of chimeric dimers" ANALYTICAL BIOCHEMISTRY, vol. 300, no. 2, 15 January 2002 (2002-01-15), pages 185-191, XP002338942 ISSN: 0003-2697

## Description

The present invention relates to an expression system for preparing nitrile hydratases. Nitrile hydratases consist of different subunits. The present system makes it possible to prepare nitrile hydratases in a manner superior to that of the prior art by expressing the nucleic acid sequences encoding these subunits separately on separate plasmids.

The amide and carboxylic acid structural classes are becoming increasingly important as precursors of fine chemicals. Special aminoamides and (proteinogenic and nonproteinogenic) amino acids are key intermediates for synthesizing pharmaceutical and agrochemical products as well as in the foodstuff field. Enantiomerically pure amides and amino acids, in particular, play a role which is becoming ever more important in the abovementioned application fields.

Aminonitrile precursors, as are required for preparing the abovementioned compound classes, can readily be obtained in racemic form using the Strecker synthesis. The nitriles which are obtained in this way can then be converted by means of chemical or enzymic hydrolysis into the corresponding amides and carboxylic acids.

Three enzymes which can participate in the enzymic hydrolysis of nitriles are known. Nitrilases convert a nitrile function directly into the acid, whereas nitrile hydratases (E.C. 4.2.1.84) form the corresponding amide under these circumstances. This latter can finally be converted into the corresponding carboxylic acid by an amidase (E.C. 3.5.1.4) (scheme 1).

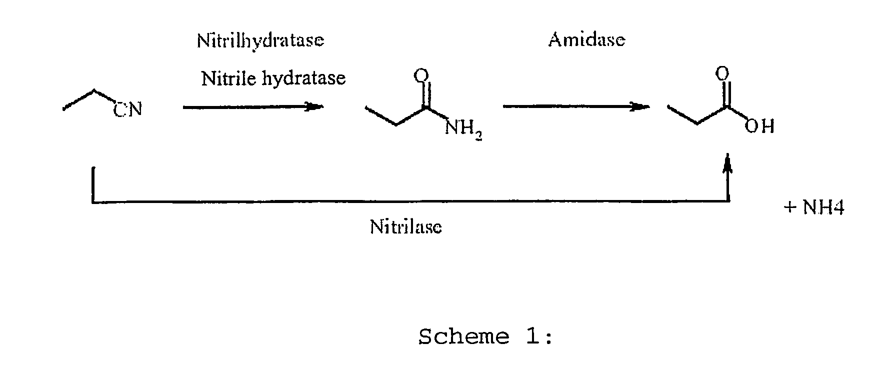

Hydrolyzing nitriles to the corresponding amides and acids using isolated enzymes or whole-cell catalysts helps to save large quantities of salt which would otherwise accrue in connection with the neutralization step following the chemical hydrolysis of nitriles. For this reason, the enzymic hydrolysis of nitriles to, for example, aminoamides and/or amino acids constitutes a more sustainable production process.

In their active form, nitrile hydratases consist of 2 nonhomologous α- and β-subunits. These subunits form heterodimers and tetramers, while decamers have even been demonstrated in the case of *Rhodococcus rhodochrous* J1. While the α- and β-subunits are of approximately the same size, they are otherwise in no way similar to each other. Nitrile hydratases are metalloproteins which contain Fe³⁺ or Co³⁺ (Bunch A.W. (1998), Nitriles, in: Biotechnology Volume 8a, Biotransformations I, Chapter 6, Eds.: Rehm HJ, Reed G, Wiley-VCH, p. 277-324; Shearer J, Kung IY, Lovell S, Kaminsky W, Kovacs JA (2001) Why is there a "inert" metal center in the active site of nitrile hydratase? Reactivity and ligand dissociation from a five-coordinate Co(III) nitrile hydratase model. J Am Chem Soc 123: 463-468; Kobayashi M, Shimizu S (2000) Nitrile hydrolases. Current Opinion in Chemical Biology 4: 95-102).

One of the greatest challenges to date has been that of heterologously preparing nitrile hydratases in a suitable host, preferably in *E. coli.* This Gram-negative bacterium is known for its ability to express heterologous proteins at high rates. Another advantage is the yield of biomass in high-cell-density fermentations using *E. coli.* In this connection, it is possible to achieve productivities of more than 100 g of dry biomass (DBM) in from 24 to 44 hours (Lee SY (1996) High cell-density culture of Escherichia coli. TIBTECH 14:98-105; Riesenberg D, Guthke R (1999) High-cell-density cultivation of microorganisms. Appl Microbiol Biotechnol 51:422-430).

Most of the sequences for the nitrile hydratase α- and β-subunits are known from the genus Rhodococcus. However, it is precisely this genus whose nitrile hydratases have thus far only been expressed in *E. coli* with particular difficulty (Ikehata O, Nishiyama M, Horinouchi S, Beppu T (1989) Primary structure of nitrile hydratase deduced from the nucleotide sequence of a Rhodococcus species and its expression in Escherichia coli. Eur J Biochem 181: 563-570).

The literature describes one-vector systems for expressing nitrile hydratases, the specific activities of which systems lie between 4.2 and 12.2 U/mg of total protein in the case of codependent *R. rhodochrous* J1 nitrile hydratases (Kobayasjhi M, Nishiyama M, Nagasawa T, Horinouchi S, Beppu T, Yamada H (1991) Cloning, nucleotide sequence and expression in Escherichia coli of two cobalt-containing nitrole hydratase genes from Rhodococcus rhodochrous. Biochim Biophys Acta 1129: 23-33) and 452 U/mg of total protein in the case of an iron-dependent *Rhodococcus spec.* N-771 nitrile hydratase (Njori M, Yohda M, Odaka M, Matsushita Y, Tsujimura M, Yoshida T, Dohmae N, Takio K Endo I (1999) Functunal expression of Nitrile hydratases in E. coli: Requirement of a nitrile hydratase activator and a post-translational modification of a ligand cysteine. J Biochem 125: 696-704), which corresponds roughly to approx. 248 U/mg of DBM (dry biomass) (calculation in accordance with Goodsell DS (1991) Inside a cell. TIBS 16: 203-206). Interestingly, it was not possible to reproduce the latter activity with nitrile hydratases from *R. erythropolis*, which is closely related to *Rhodococcus spec.* N-711, using similar vector systems and dispositions of the structural genes. There was, therefore, still a need for processes and systems which permit the enzymes under consideration to be made available in a manner which is adequate for industrial scale preparations.

The skilled person is already familiar with the use of two-vector expression systems for heterologously expressing recombinant proteins in *E. coli,* for example for forming the motor protein kinesin (Skowronek K, Kasprzak A (2002) A two-plasmid system for independent genetic manipulation of subunits of homodimeric proteins and selective isolation of chimeric dimers. Analytical Biochemistry 300: 185-191), the plasminogen proactivator streptokinase (Yazdani SS, Mukherjee KJ (2002) Continuous-culture studies on the stability and expression of recombinant streptokinase in Escherichia coli; stability and expression of streptokinase in continuous culture. Bioprocess and Biosystems Engineering 24(6): 341-346), the complex of two human proteins (hematopoietic cell tyrosine phosphatase and mitogen protein kinase; Kholod N, Mustelin T (2001) Novel vectors for coexpression of two proteins in E. coli. 31: 322-328) or the human CKMB creatine kinase (WO95/12662).

However, no heteromeric enzymes, such as the nitrile hydratases, which are used as biocatalysts in the chemical industry have thus far been expressed using such a system.

The object was, therefore, to develop an expression system which makes it possible to efficiently express both cobalt-dependent and iron-dependent nitrile hydratases actively in *E*. *coli*. In particular, the system according to the invention should be able to make the enzymes under consideration available at a rate of expression which is higher, and, where appropriate, in forms which are more stable, than in the case of the prior art in order, in this way, to make the use of these enzymes on an industrial scale advantageous from ecological and economic points of view.

These objects, and other objects which are not specified in detail but which ensue in an obvious manner from the prior art, are achieved by specifying an expression system having the features of the present claim 1. Claims 2 to 8 relate to preferred embodiments of the expression system according to the invention. Claims 9 and 10 are directed towards processes for preparing nitrile hydratases and/or (amino)carboxylic acids or (amino)carboxamides. Claim 11 protects a host organism which is equipped with the expression system.

The set object is achieved, extremely advantageously and nonetheless entirely surprisingly, by, in the case of an expression system for simultaneously expressing the nucleic acid sequences encoding the different subunits of a nitrile hydratase, the expression system possessing in each case at least one plasmid containing at least one nucleic acid sequence encoding the respective subunit. Using the proposed expression system, it is possible to heterologously express the nucleic acid sequences under consideration in a manner which is adequate for industrial scale preparations. In this connection, it may be particularly surprising that simply expressing the nucleic acid sequences encoding the corresponding nitrile hydratase subunits, which nucleic acid sequences are in fact organized in one operon, separately on different plasmids contributes to increasing the activity of the resulting nitrile hydratases by a factor of > 8 as compared with the "normal" expression. It was not possible to deduce this in an obvious manner from the prior art.

The expression system according to the invention can be used in all the host organisms which the skilled person takes into consideration for the present purpose. Microorganisms which are to be mentioned in this regard are organisms such as yeast, such as *Hansenula polymorpha, Pichia sp.* and *Saccharomyces cerevisiae,* prokaryotes, such as *E. coli* and *Bacillus subtilis,* or eukaryotes, such as mammalian cells, insect cells or plant cells. Host organisms into which plasmids containing the nucleic acid sequences can be cloned are used for replicating and isolating a sufficient quantity of the recombinant enzyme. The methods used for this purpose are well known to the skilled person (Sambrook, J.; Fritsch, E.F. and Maniatis, T. (1989), Molecular cloning: a laboratory manual, 2nd ed., Cold Spring Harbor Laboratory Press, New York). Preference is given to using E. *coli* strains for this purpose. The following are very particularly preferred: E. coli XL1 Blue, NM 522, JM101, JM109, JM105, RR1, DH5α, TOP 10-, HB101, BL21 codon plus, BL21 (DE3) codon plus, BL21, BL21 (DE3) and MM294. Plasmids which are preferably used to clone the gene construct containing the nucleic acid according to the invention into the host organism are likewise known to the skilled person (see also PCT/EP03/07148; see below). Very particular preference is given to an expression system which is present in E. *coli* BL21 as the host.

Promoters are DNA sequence regions from which transcription of a gene or operon is controlled. The promoters which are particularly advantageous for implementing the invention and which are to be used, in particular, in *E. coli* are known to the skilled person (Sambrook, J.; Fritsch, E.F. and Maniatis, T. (1989), Molecular cloning: a laboratory manual, 2nd ed., Cold Spring Harbor Laboratory Press, New York). It has now proved to be advantageous if the expression of the nucleic acid sequences encoding the subunits is in each case under the control of the same promoter so that the nucleic acid sequences encoding the subunits can be expressed at a rate which is as identical as possible. Suitable promoters can be selected from the group T7, lac, tac, trp, ara or rhamnose-inducible. Other promoters are mentioned in (Cantrell, SA (2003) Vectors for the expression of recombinant proteins in E. coli. Methods in Molecular biology 235: 257-275; Sawers, G; Jarsch, M (1996) Alternative principles for the production of recombinant proteins in Escherichia coli. Applied Microbiology and Biotechnology 46(1): 1-9). Very particular preference is given to using the T7 promoter in the expression system according to the invention (Studier, W.F.; Rosenberg A.H.; Dunn J.J.; Dubendroff J.W.; (1990), Use of the T7 RNA polymerase to direct expression of cloned genes, Methods Enzymol. 185, 61-89; or brochures supplied by the companies Novagen or Promega).

It has been found to be useful, for the ability of the expression system according to the invention to function, for particular nucleic acid sequences, which encode peptide sequences known to be helper proteins and whose functions have previously to a large extent been unknown, to be present on the corresponding plasmids. These sequences are known to the skilled person in regard to producing active nitrile hydratases (Nojiri M; Yohda M; Odaka M; Matsushita Y; Tsujimura M; Yoshida T; Dohmae N; Takio K; Endo I (1999) Functional expression of nitrile hydratase in Escherichia coli: requirement of a nitrile hydratase activator and post-translational modification of a ligand cysteine. Journal of biochemistry 125(4): 696-704). Very particular preference is given to an expression system in which at least one nucleic acid sequence encoding such a helper protein, in particular the p47K protein (Seq. ID No. 33) or the p12K protein (Seq. ID No. 31), is present per plasmid set employed. In this connection, a plasmid set denotes the plasmids which are required in accordance with the invention for constructing an active nitrile hydratase.

As explained in detail at the outset, nitrile hydratases are known from a variety of organisms (see also PCT/EP04/00338; Dissertation, see above). However, preference is given to using, in the expression system according to the invention, those nucleic acid sequences which encode nitrile hydratase subunits which have their origin in nitrile hydratases derived from Rhodococcus strains. In this connection, the nucleic acid sequences which are employed can be altered, as compared with the original sequences from Rhodococcus, by means of mutagenesis on a chemical or molecular biological basis. In this connection, particular consideration is given to those nucleic acid sequences which encode subunits which are improved, as compared with the wild-type sequences, in regard to activity and/or selectivity and/or stability. According to the invention, the improvement in the activity and/or selectivity and/or stability denotes that the enzymes under consideration are more active and/or more selective or less selective, or more stable under the reaction conditions employed. While the activity and the stability of the enzymes should naturally, for the industrial application, be as high as possible, the selectivity is considered to be improved when either the substrate selectivity decreases but the enantioselectivity of the enzymes is increased.
The skilled person is sufficiently familiar with the procedure for using mutagenesis methods to improve the nucleic acid sequences according to the invention or the polypeptides which they encode. The mutagenesis methods which are suitable are any methods which are available to the skilled person for this purpose. In particular, these methods are saturation mutagenesis, random mutagenesis, in-vitro recombination methods and site-directed mutagenesis (Eigen, M. and Gardiner, W. (1984), Evolutionary molecular engineering based on RNA replication, Pure Appl. Chem. 56, 967-978; Chen, K. and Arnold, F. (1991), Enzyme engineering for nonaqueous solvents: random mutagenesis to enhance activity of subtilisin E in polar organic media. Bio/Technology 9, 1073-1077; Horwitz, M. and Loeb, L. (1986), Promoters Selected From Random DNA-Sequences, Proc Natl Acad Sci USA 83, 7405-7409; Dube, D. and L. Loeb (1989), Mutants Generated By The Insertion of Random Oligonucleotides Into The Active-Site of The Beta-Lactamase Gene, Biochemistry 28, 5703-5707; Stemmer, P.C. (1994), Rapid evolution of a protein in vitro by DNA shuffling, Nature 370, 389-391 and Stemmer, P.C. (1994), DNA shuffling by random fragmentation and reassembly: In vitro recombination for molecular evolution. Proc Natl Acad Sci USA 91, 10747-10751).
Particular preference is given to the nucleic acid sequences encoding the nitrile hydratase subunits being derived from Rhodococcus strains, in particular *R. erythropolis* 870-AN019.

In another preferred embodiment, the nucleic acid sequences employed are altered such that they correspond particularly well to the E. *coli* codon usage. It has been found that the yields of the enzymes obtained can be increased still further in proportion to the extent to which the codon usage of the gene to be expressed corresponds to that of E. *coli.* Particular preference is therefore given to modifying the nucleic acid sequences which encode the nitrile hydratase subunits in conformity with the *E. coli* codon usage. "Codon usage" is understood as being the fact that different organisms use different base triplets, which encode the same amino acids (degeneracy of the genetic code) to differing extents.

In principle, the plasmids or vectors which can be used are any types which are available to the skilled person for this purpose. These plasmids and vectors are listed, for example, in Studier and coworkers (Studier, W.F.; Rosenberg A.H.; Dunn J.J.; Dubendroff J.W.; (1990), Use of the T7 RNA polymerase to direct expression of cloned genes, Methods Enzymol. 185, 61-89) or the brochures supplied by the companies Novagen, Promega, New England Biolabs, Clontech and Gibco BRL. Other preferred plasmids and vectors can be found in: Glover, D.M. (1985), DNA cloning: a practical approach, Vol. I-III, IRL Press Ltd., Oxford; Rodriguez, R.L. and Denhardt, D.T. (eds) (1988), Vectors: a survey of molecular cloning vectors and their uses, 179-204, Butterworth, Stoneham; Goedeel, D.V. (1990), Systems for heterologous gene expression, Methods Enzymol. 185, 3-7; Sambrook, J.; Fritsch, E.F. and Maniatis, T. (1989), Molecular cloning: a laboratory manual, 2nd ed., Cold Spring Harbor Laboratory Press, New York. Plasmids which can be used to clone the gene constructs containing the nucleic acid sequences encoding the subunits into the host organism in a very preferred manner are: pUC18/19 (Roche Biochemicals), pKK-177-3H (Roche Biochemicals), pBTac2 (Roche Biochemicals), pKK223-3 (Amersham Pharmacia Biotech), pKK-233-3 (Stratagene) and pET (Novagen). Very particular preference is given to an expression system which is based on plasmids belonging to the pET series. Extreme preference is given to using plasmids of the same series for expressing both the nucleic acid sequence encoding the α subunit and the nucleic acid sequence encoding the β subunit.

In another embodiment, the present invention relates to a method for preparing nitrile hydratases. The method is characterized in that it is carried out using an expression system according to the invention as described above.

In a preferred embodiment, the method according to the invention is carried out such that the expression is performed at incubation temperatures of less than 30 degress Celsius, preferably less than 25 degrees Celsius and very particularly preferably at ≤ 20 degrees Celsius. The embodiment in which alcohols, in particular ethanol, are added to the medium, during the expression, at a concentration of less than 10% (w/w), preferably less than 5% (w/w) and very particularly preferably 2-4% (w/w) is also advantageous. Implementing these measures results in insoluble proteins (inclusion bodies), which do not display any activity, either not being formed, or only being formed to a decreased extent, in the method according to the invention for preparing nitrile hydratases.

In another embodiment, the present invention relates to a host organism which possesses an expression system according to the invention. As already indicated above, the nucleic acid sequences encoding the nitrile hydratase subunits can, in a manner according to the invention as described above, be integrated into plasmids and transformed into host organisms. In addition, and as well as the expression system according to the invention, cloned genes for an amidase which may work stereoselectively (e.g. the amidase disclosed in WO2004/005517 or EP 1318193) may also be present in the host organism. A whole-cell catalyst which has been prepared in this way is advantageously able to produce both the enzymes involved in the nitrile breakdown, thereby ensuring that the nitrile employed is immediately converted into the corresponding carboxylic acid. Whole-cell catalysts which comprise several enzymes which are involved in a reaction cascade have already been disclosed (EP1216304). They are used in the present invention in an equivalent manner. If rhamnose-inducible promoters are used, an organism as specified in DE10155928 should then be employed as the host organism or whole-cell catalyst. It is furthermore advantageous to use an *E. coli* BL21 codon plus which may have been modified in an equivalent manner to that described in DE10155928.
In order to match the expression of the enzymes with regard to their turnover rates, the respective nucleic acid sequences encoding the nitrile hydratase and the amidase can, in accordance with their turnover rates, be cloned in different plasmids having different copy numbers and/or promoters of differing strength in order to obtain differing strengths of expression of the nucleic acid sequences. In enzyme systems which are matched in this way, there is advantageously no accumulation of an intermediate compound, which may have an inhibitory effect, and the reaction under consideration can take place at an optimal overall rate. However, this is sufficiently well-known to the skilled person (Gellissen, G.; Piontek, M.; Dahlems, U.; Jenzelewski, V.; Gavagan, J.W.; DiCosimo, R.; Anton, D.L.; Janowicz, Z.A. (1996), Recombinant Hansenula polymorpha as a biocatalyst. Coexpression of the spinach glycolate oxidase (GO) and the S. cerevisiae catalase T (CTT1) gene, Appl. Microbiol. Biotechnol. 46, 46-54; Farwick, M.; London, M.; Dohmen, J.; Dahlems, U.; Gellissen, G.; Strasser, A.W.; DE19920712).
The skilled person is likewise familiar with preparing such whole-cell catalyst (Sambrook, J.; Fritsch, E.F. and Maniatis, T. (1989), Molecular cloning: a laboratory manual, 2nd ed., Cold Spring Harbor Laboratory Press, New York; Balbas, P. and Bolivar, F. (1990), Design and construction of expression plasmid vectors in E. coli, Methods Enzymol. 185, 14-37; Rodriguez, R.L. and Denhardt, D.T. (eds) (1988), Vectors: a survey of molecular cloning vectors and their uses, 205-225, Butterworth, Stoneham).

Another embodiment of the present invention relates to a method for preparing (amino)carboxylic acids or (amino)carboxamides which may, where appropriate, be enantiomer-enriched. This method is also carried out using a host organism or an expression system as described above. This means that the nitrile hydratases which are involved in the preparation of the carboxylic acids or carboxamides are obtained using a host organism as has just been described. For this application, the enzymes under consideration can be used in free form, as homogeneously purified compounds, or as recombinantly (rec-) prepared enzymes. Furthermore, the enzymes can also be used as components of an intact guest organism or in combination with the disrupted and, if desired, highly purified cell mass of the host organism.

It is likewise possible to use the enzymes in immobilized form (Sharma B.P.; Bailey L.F. and Messing R.A. (1982), Immobilisierte Biomaterialiern - Techniken und Anwendungen [Immobilized biomaterials - techniques and applications], Angew. Chem. 94, 836-852). The immobilization is advantageously effected by lyophiLization (Paradkar, V.M.; Dordick, J.S. (1994), Aqueous-Like Activity of -Chymotrypsin Dissolved in Nearly Anhydrous Organic Solvents, J. Am. Chem. Soc. 116, 5009-5010; Mori, T.; Okahata, Y. (1997), A variety of lipi-coated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solbilize the enzyme in active form in toluene, Biocatalysis 6, 291-305). Very particular preference is given to the lyophilization being carried out in the presence of surfactants such as Aerosol OT or polyvinylpyrrolidone or polyethylene glycol (PEG) or Brij 52 (diethylene glycol monocetyl ether) (Kamiya, N.; Okazaki, S.-Y.; Goto, M. (1997), Surfactant-horseradish peroxidase complex catalytically active in anhydrous benzene, Biotechnol. Tech. 11, 375-378).
Extreme preference is given to immobilizing on Eupergit^{®}, in particular Eupergit C^{®} and Eupergit 250L^{®} (Röhm) (Eupergit.RTM. C, a carrier for immobilization of enzymes of industrial potential. Katchalski-Katzir, E.; Kraemer, D.M. Journal of Molecular Catalysis B: Enzymatic (2000), 10(1-3), 157-176).
Preference is also given to immobilizing on Ni-NTA in combination with the His tag (hexahistidine)-supplemented polypeptide (Purification of proteins using polyhistidine affinity tags. Bornhorst, Joshua A.; Falke, Joseph J. Methods in Enzymology (2000), 326, 245-254). The use as CLECs is likewise conceivable (St. Clair, N.; Wang, Y.-F.; Margolin, A.L. (2000), Cofactor-bound cross-linked enzyme crystals (CLEC) of alcohol dehydrogenase, Angew. Chem. Int. Ed. 39, 380-383).
Using these measures, it is possible to generate polypeptides (enzymes) which are stable, and can operate, in mixtures of aqueous and organic solvents, or in organic solvents on their own, from equivalent polypeptides which are rendered unstable by organic solvents.

The following strains are used in the experiments which are described below:
List of the strains employed. (Brandão PFB, Clapp JP and Bull AT (2002). Discrimination and taxonomy of geographically diverse strains of nitrile-metabolising actinomycetes using chemometric and molecular sequencing techniques. Environmental Microbiology 4, 262-276; see also PCT/EP04/00338).

**Table 1:**

| Isolate | DSM | α-subunit Seq. No. | β-subunit Seq. No. |
|---|---|---|---|
| R. erythropolis 870-AN019 | 15258 | 1 | 3 |
| R. erythropolis ENG-AN033 | 15261 | 5 | 7 |
| R. erythropolis 871-AN042 | 15265 | 9 | 11 |
| *R. rhodochrous* M8 | Russian National Collection of Microorganisms VKPM-S-926 | 13 | 15 |

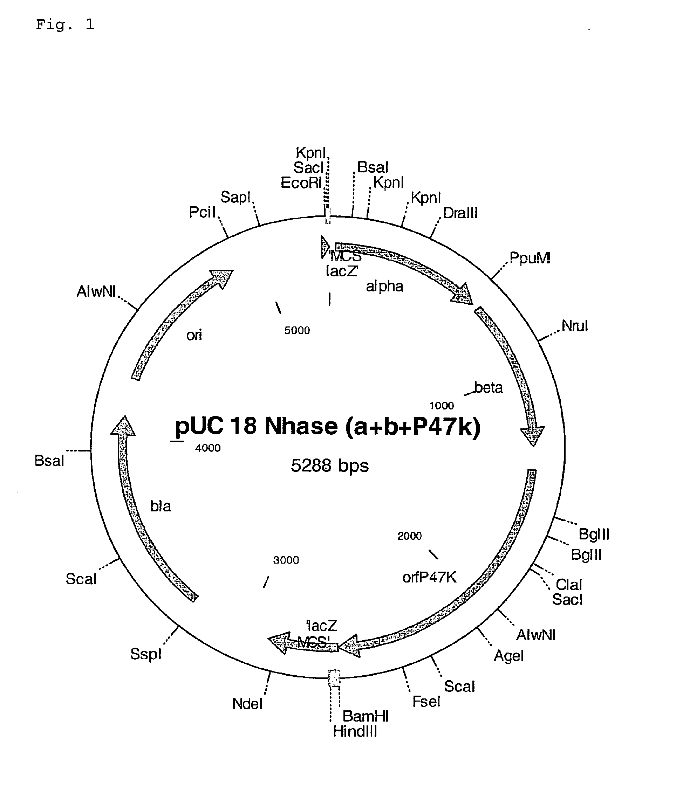

The following primers were used for the blunt-end cloning in pUC18/19 (xSmaI) (fig. 1) of the nitrile hydratases and the p47K protein (Seq. ID No. 33) from the abovementioned strains:

| Primer | Primer sequence | Amplified Subunits | Seq. No. |
|---|---|---|---|
| NH-Re-N | 5'-GCC CGC ATA AGA AAA GGT GAA C | α, β, p47K | 17 |
| NH-Re-C-p47K | 5'-GCA TGC CTT CAA ATC AGC CTC | α, β, p47K | 18 |

The first expression experiments in a one-vector expression system for nitrile hydratases from the *R. erythropolis* strains 870-AN019, 871-AN042 and ENG-AN033 were carried out using plasmids of the pUC18/19 series in different *E. coli* strains. In order to be able to identify the optimal expression host, the pUC18/19 constructs containing the nitrile hydratases from *R. erythropolis* strains 870-AN019, 871-AN042 and ENG-AN033 were transformed into different *E. coli* strains and expressed under the control of the *lac* promoter. The *E. coli* strains in this context were the following: JM109, DM5α, BL21 codon plus, BL21, HB101, MM294 and XL1blue.

The activities (units per g of dry cell mass) of the recombinant nitrile hydratases in the different hosts can be summarized as follows: it was found that the highest activity was obtained with *E. coli* BL21 codon plus RIL (from Stratagene) in which the copy numbers of the t-RNAs for arginine (AGA/AGG), isoleucine (AUA) and leucine (CUA), which t-RNAs are rare in the case of *E. coli,* are increased.

This host organism was constructed in order, in particular, to express genes having a codon usage which is adapted to a high GC content, for example the genes from rhodococci (72% GC).

The *R*. *erythropolis* 870-AN019 nitrile hydratase expressed in *E. coli* BL21 codon plus shows by far the highest activity, of 100 U/g of DBM, with this being followed by 870-AN019 nitrile hydratases expressed in DL5α (8 U/g- of DBM) and ENG-AN033 nitrile hydratases expressed in BL21 codon plus (7 U/g of DBM).
The activities of all the other recombinant organisms were less than 2 U/g of DBM and were undetectable. An activity of 280 U/g of DBM was even achieved for the 870-AN019 nitrile hydratase under optimized conditions.

In that which follows, iron-dependent nitrile hydratases were expressed in a two-vector expression system, with α and β subunits each being present on a separate plasmid. The advantage of this.system is that the two units are in each case directly under the control of the T7 promoter, which is employed, where appropriate, and the transcripts for the two genes are consequently formed to equal extents. The p47K helper protein (Seq. ID No. 33) was located downstream of one of the two subunits on a case-by-case basis. Stratagene plasmids of the pET series (pET22b and pET26b) were used as expression vectors. The aim was to express *R. erythropolis* 870-AN019 nitrile hydratase (Seq. Nos. 1 and 3).

The following primers were used for cloning the two subunits and the p47K protein (Seq ID No. 33):

| Primer | Primer sequence | Amplified orf | Seq. No. |
|---|---|---|---|
| NH019-α-for-Nde | | α | 19 |
| NH019-α-rev-Bam | | α | 20 |
| NH019-β-for-Nde | | β | 21 |
| NH019-β-rev-Eco | | β | 22 |
| NH019-p47K-for-Bam | | p47K | 23 |
| NH019-p47K-rev-Hind | | p47K | 24 |

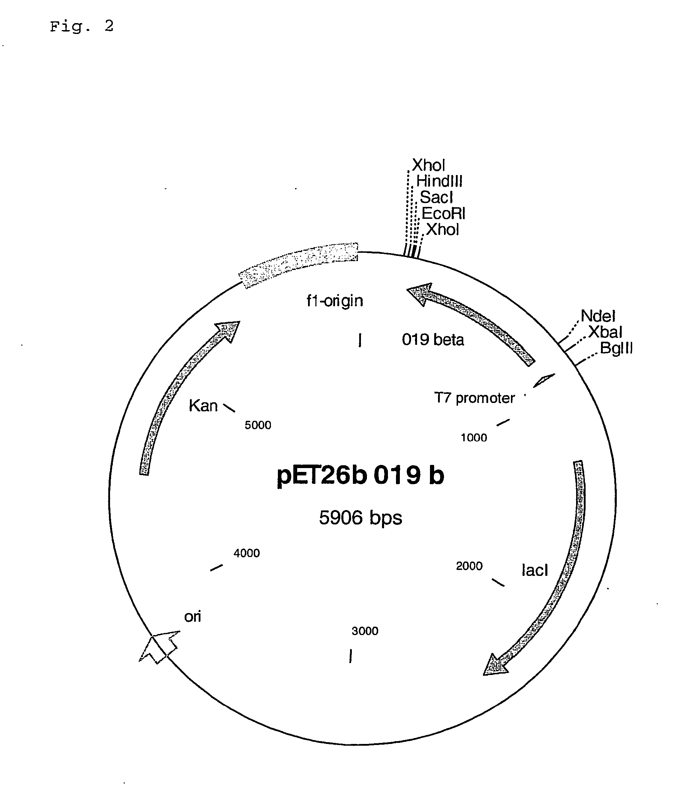

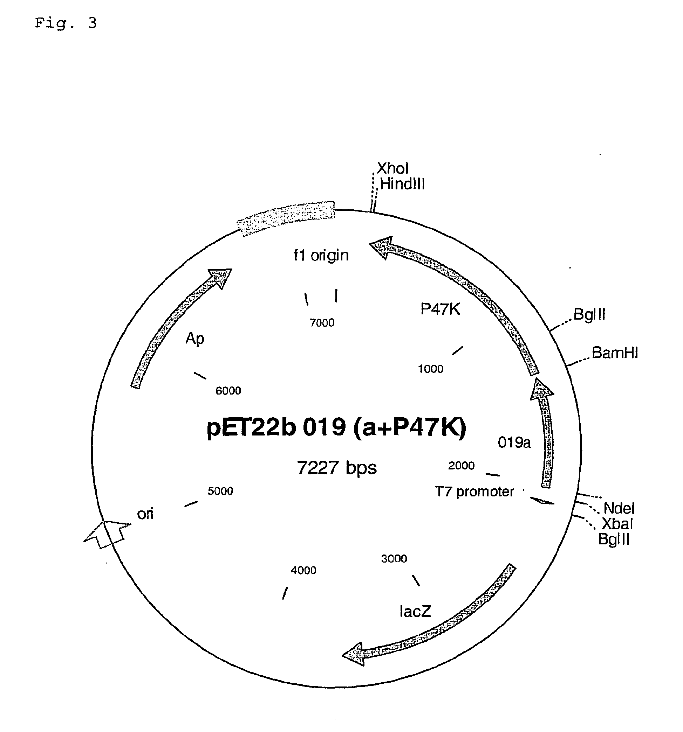

Primers which were derived from the *R. erythropolis* 870-AN019 nitrile hydratase sequence (Brandao PFB, Clapp JP, Bull AT (2003) Diversity of nitrile hydratase and amidase enzyme genes in Rhodococcus erythropoli recovered from geographically distinct habitats. Applied and Environmental Microbiology 69(10): 5754-5766; see also PCT/EP04/00338), and which were provided with cleavage sites for the restriction endonucleases *Nde*I (N-terminally) and, respectively, *Bam*HI (α subunit) or EcoRI (β subunit) C-terminally, were used for the α and β subunits. The primers for p47K were likewise derived from this organism and contained the cleavage sites for *Bam*HI (N-terminally) and, respectively, *Hin*DIII (C-terminally). The β subunit was cloned into pET26b while the α subunit and p47K were together cloned into pET22b. This resulted in the following plasmids, which were transformed into E. coli BL21 codon (+) RIL (see also figs. 2/3).

The activities of the *R. erythropolis* 870-AN019 nitrile hydratase in the *E. coli* strains BL21 and BL21 codon plus RIL (see above) were prepared. When using the above-described expression system, activities of approx. 1750 U/g of DBM and of 2750 U/g of DBM (based on benzonitrile as substrate) were achieved when using *E. coli* BL21 and *E. coli* BL21 codon (+), respectively. This signifies an increase by a factor of from 5.3 to 8.3 as compared with the one-vector expression systems.

However, it was true that 50% of the recombinant protein in the cell was present, in these experiments, as what are termed inclusion bodies. In order to further improve the activities of the nitrile hydratases, parameters such as IPTG concentration, temperature during the heterologous expression, and the supply of additives to the medium, were investigated. Reducing the IPTG concentration had no effect on the solubility of the recombinant nitrile hydratases. All the subsequent experiments were therefore carried out using 1 mM IPTG. Lowering the incubation temperature had the greatest effect in reducing the formation of inclusion bodies.
By reducing the temperature down to 20°C after the heterologous expression had been induced, it was possible to transfer a large proportion of the enzyme into the soluble fraction. Adding 3% ethanol to the medium had a further positive effect. Under these conditions (1 mM IPTG, 20°C incubation temperature, 3% added ethanol) it was possible to achieve an activity of 6480 U/g of DBM.
A further increase in activity was achieved by using a synthetic 870-AN019 nitrile hydratase gene. The corresponding nucleic acid sequences encoding the α and β subunits were prepared while taking into consideration the *E. coli* codon usage and retaining the amino acid sequences of the two genes. The intended advantage of these synthetic genes was that the DNA sequence would be optimally adapted for expression in *E. coli* and that consequently it would also be possible to dispense with using the "codon plus" strain. The subunits were then separately cloned into pET vectors, as previously described, and expressed in *E. coli* BL21 under the above optimized conditions. Using this strain, an activity of approx. 10 000 U/g of DBM was achieved. Consequently, this strain is more than twice as active as the wild-type *R. erythropolis* 870-AN019, which has an activity of approx. 4760 U/g of DBM when using benzonitrile as substrate. Table 2 provides a summary of the activities which were achieved.

In the E. *coli* host, the synthetic nitrile hydratase constitutes approx. 50% of the total cell protein formed, with approx. 20% being present as dissolved recombinant protein.

**Table 2: Overview of the nitrile hydratase activities which were measured using different expression systems.**

| Expression system (Host & promoter) | 870-AN019 nitrile hydratase expression conditions | | |
|---|---|---|---|
| | Native subunits, standard medium, 26°C | Native subunits, medium + 3% EtOH, 20°C | Synthetic subunits medium + 3% EtOH, 20°C |
| E. coli BL21 (DE3) + T7 promoter | 1750 U/g DBM | - | 10080 U/g DBM |
| E. coli BL21 (DE3) codon usage & T7 promoter | 2750 U/g DBM | 6480 U/g DBM | - |

In conclusion, the expression of cobalt-dependent nitrile hydratases was investigated in a two-vector expression system. The cobalt-dependent *Rhodococcus rhodochrous* M8 nitrile hydratase (Pogorelva TE, Ryabchenko LE, Sunzov NI, Yanenko AS (1996) Cobalt-dependent transcription of nitrile hydratase gene in Rhodococcus rhodochrous M8. FEMS Microbiology Letters 144: 191-195) was cloned and prepared using the above-described expression system according to the invention.

The nitrile hydratase sequence (Seq. ID Nos. 13 and 15) is deposited in GenBank (DDBJ, EMBL and NCBI DNA database) under the reference X86737 and is freely available. The following primers were used for cloning the individual subunits and the *R. rhodochrous* M8 p12K protein (Seq. ID No. 31):

| Primer | Primer sequence | Amplified subunits | Seq. No. |
|---|---|---|---|
| M8-α-for-Nde | | α | 25 |
| M8-α-rev-Bam | | α | 26 |
| M8-β-for-Nde | | β | 27 |
| M8-β-rev-Bam | | β | 28 |
| M8-p12K-for-Bam | | p12K | 29 |
| M8-p12K-rev-Sac | | p12K | 30 |

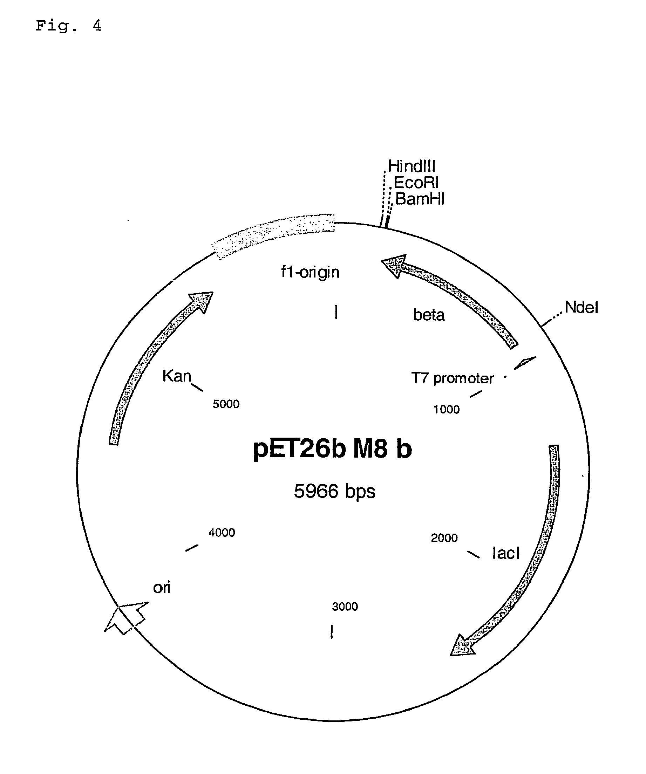

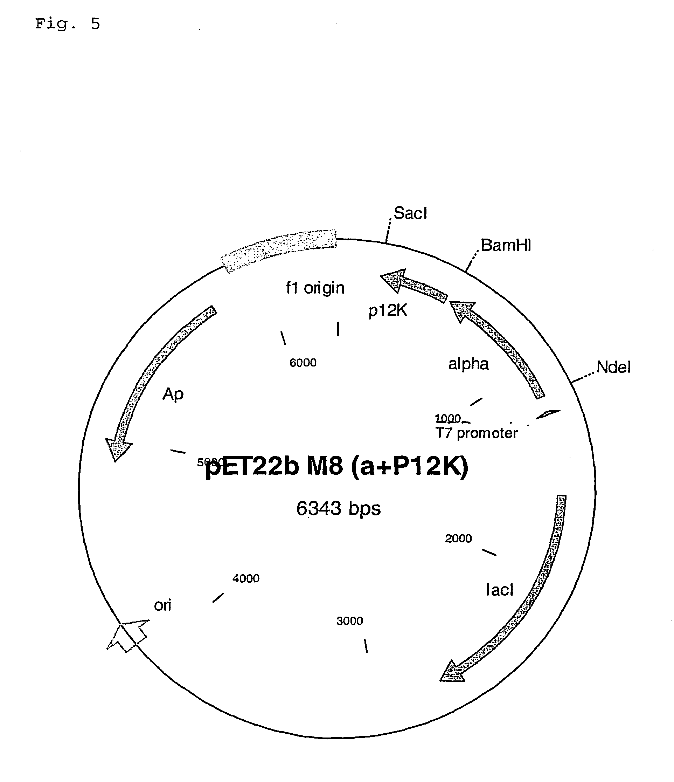

Primers which were derived from the M8 nitrile hydratase sequence and which were provided with cleavage sites for the restriction endonucleases *Nde*I (N-terminally) and, respectively, *Bam*HI (C-terminally) were used for the α and β subunits. The primers for p12K were derived from the *R. rhodochrous* J1 sequence and contained the cleavage sites for BamHI (N-terminally) and, respectively, SacI (C-terminally). The β subunit (Seq. ID No. 15) was cloned into pET26b while the α subunit (Seq. ID No. 13) and p12K (Seq. ID No. 31) were cloned into pET22b. This resulted in the following plasmids (figs. 4/5), which were transformed into *E. coli* BL21 codon(+) RIL.

In contrast to the expression of iron-dependent nitrile hydratases in *E. coli*, the cells had, in the case of the cobalt-dependent nitrile hydratase, to be precultured over several generations in order to accustom to the toxic cobalt (0.5 mM employed).

Table 3 compares the activities of the recombinant cobalt-dependent (*R. rhodochrous* M8) and iron-dependent (*R*. *erythropoli* 870-AN019) nitrile hydratases.

**Table 3: Activities of the cobalt-dependent and iron-dependent nitrile hydratases which were measured, following expression in accordance with the invention, using acrylamide as substrate.**

| Recombinant nitrile hydratase from | Activity (U/mg); substrate, acrylamide |
|---|---|
| *R. rhodochrous* M8 (Co) | 160 |
| *R. erythropolis* 870-AN019 (Fe) (synthetic, see above) | 250 |

It was thus possible to demonstrate that the expression system according to the invention can advantageously be used both for cobalt-dependent and for iron-dependent nitrile hydratases. It is possible, in particular, to achieve dramatic increases in activity when the expression systems and/or sequences are appropriately optimized. In addition, as a result of the successful separate expression of the nitrile hydratase subunits, it is possible to investigate novel combinations of subunits belonging to different nitrile hydratases, with this helping to increase the diversification of the nitrile hydratases and consequently the diversification of their properties. At the time of the invention, it was not possible to deduce this, in an obvious manner, from the prior art.

Within the context of the invention, "optically enriched (enantiomerically enriched, enantiomer-enriched) compounds" are understood as meaning the presence of one optical antipode in a mixture with the other at a concentration of > 50 mol%.

The term "nucleic acid sequences" subsumes all types of single-stranded or double-stranded DNA as well as RNA, or mixtures thereof.

The organisms 870-AN019, ENG-AN033 and 871-AN042 were deposited by the applicant, on 10.22.2002, in the Deutsche Sammlung für Mikroorganismen und Zellculkuren [German collection of microorganisms and cell cultures], Mascheroder Weg 4, 38124 Braunschweig in accordance with the Budapest treaty. The organism *Rhodococcus rodochrous* M8 is deposited in the all-Russian national collection of microorganisms under number VKPM-S-926. The corresponding sequences are contained in the gene database (see above).

According to the invention, the term "expression system" is understood such that it means nucleic acid-based biological material which is able, in organisms, to bring about the expression of the nucleic acid sequences which are inherent to it and which encode the nitrile hydratase subunits. This biological material comprises, in particular, plasmids and vectors.

The expressions "protein" and "polypeptide" are used synonymously within the context of the invention.

### Description of the figures:

Figure 1: The vector map shows the general disposition of the nitrile hydratase α and β subunits and, respectively, the p47K orf in the pUC18 plasmid in an expression system using one vector.
Figures 2/3: The vector maps show the disposition of the nitrile hydratase α and β subunits and, respectively, the p47K orf from *R. erythropolis* 870-AN019 in plasmids of the pET series in an expression system using two vectors.
Figures 4/5: The vector maps show the disposition of the α and β subunits and, respectively, the p12K orf from *R*. *rhodochrous* M8 in plasmids of the pET series in an expression system using two vectors.

### Experimental section:

### Culturing microorganisms

The E. coli cells are cultured and stored as described in Sambrook et al. (Sambrook, J.; Fritsch, E.F. and Maniatis, T. (1989), Molecular cloning: a laboratory manual, 2nd ed., Cold Spring Harbor Laboratory Press, New York).

A chelate mineral medium (CMM) as described by Heald et al. 2001 (Heald S.C., P.F.B. Brandao, R. Hardicre and A.T. Bull, 2001: Physiology, biochemistry and taxonomy of deep-sea nitrile metabolising Rhodococcus strains. Antonie van Leeuwenhoek 80, 169-183) was used for culturing rhodococci. The CM medium is supplemented with 5 g of glucose/l. 2.4 mg of CoCl₂^{*} 6 H₂O/l are used for strains containing cobalt-dependent enzymes while 50 mg of FeSO₄^{*} 7 H₂O/l are used for strains containing iron-dependent enzymes.

The samples removed from the culture were diluted with potassium phosphate buffer used for the culturing (6 ml of a 200 g/l K₂HPO₄ solution/l and 4 ml of a 157.5 g/l KH₂PO₄ solution/l) such that the measurement range was between 0.05 and 0.3. The buffer served as the reference. Measurements were made at 600 nm.

### PCR

For amplifying DNA fragments from rhodococci, the following components were pipetted in per 50 µl assay:
100 ng of chromosomal DNA
1 µl of dNTP mix (in each case 10 mM)
5 µl of buffer
2 µl of DMSO
0.5 µl of herculase (2.5 U)
H₂O to 50 µl

The thermocycler was programmed as follows:
A. 3 min 98°C
B. 40 sec 98°C
C. 40 sec X°C
D. Y min 72°C
E. 5 min 72°C
F. 4°C

Steps B, C and D were repeated 30 times. The annealing temperature X (C) is calculated from the melting temperature of the primers employed and the period of incubation Y (D) is calculated from the length of the gene to be amplified (rule, 1 kb of DNA equals 1 minute).

### Digesting with restriction enzymes

The DNA to be cut is provided with 5 U of restriction enzyme and the appurtenant buffer and, if nothing else is required, incubated at 37°C. Chromosomal DNA is digested using 10 U of enzyme. The incubation period is from 1.5 to 2.5 hours.

### Treating with alkaline phosphatase

In order to prevent vectors which have been cut with only one restriction endonuclease from religating with themselves, the protruding phosphate radical at the 5' end is removed with alkaline phosphatase. Circular DNA can only be reformed by inserting a DNA fragment.

The vector which has been cut with a restriction endonuclease is incubated at 65°C for 15 min in order to stop the restriction endonuclease. The dephosphorylation buffer is then added and the mixture is incubated, at 37°C for 10 min, with 1 U of shrimp alkaline phosphatase. The enzyme is separated off by subsequently subjecting the vector DNA to gel electrophoresis.

### Treating with T4 DNA ligase

Vector and insert are used for the ligation in a ratio of 1:3. The volume is kept as low as possible (7-20 µl). The mixture is incubated overnight at 16°C in ligation buffer and in the presence of 1 U of ligase.

### Transforming

100 µl of competent cells are pipetted in to the ligation mixture and the whole is mixed by repeatedly drawing up into the pipette. After a 30 min incubation on ice, a heat-shock step is carried out at 42°C for 45 sec and the mixture is incubated on ice for a further 2 min. 120-900 µl of SOC medium are added and the mixture is incubated at 37°C for 45 min while being shaken. The mixture is subsequently plated out and incubated overnight at 37°C.

### Expressing the different nitrile hydratases (table 1) in a one-vector expression system

The following protocol was used for the expression: 50 ml of LBₐₘₚ₁₀₀ medium containing 2 mM Fe citrate were inoculated to a concentration of 1% with an overnight culture. After an OD₆₀₀ of approx. 0.5 had been reached, expression of the nitrile hydratases in the different *E. coli* strains was induced with 1 mM IPTG (isopropylthiogalactoside). The cells were harvested approx. 24 hours after induction. The nitrile hydratases were expressed constitutively in strain DH5α since this strain does not overexpress the *lac* repressor. As a result, the step of inducing with IPTG is dispensed with in the case of this strain.

### Activity using benzonitrile as substrate:

The biotransformation was carried out on a 10 ml scale using approx. 100 mg of moist biomass (OD₆₀₀ = 5) in the potassium phosphate buffer (100 mM) pH 7.0. The incubation took place at 30°C and the substrate concentration was approx. 5 mM benzonitrile. Samples were removed every 5-10 min over a period of at most 1 hour. The sample volume was 100 µl and the reaction was stopped by adding 10 µl of 50% phosphoric acid.

The concentrations of benzonitrile and benzamide were then determined by means of HPLC:
Column: RP18 Phenomenex Hypersil ODS 5 µ column (with precolumn)
Mobile phase: 10 mM K2HPO4 (pH 2.3)
Flow rate: 1 ml/min
Wavelength: 202 nm
Injection volume: 20 µl
Duration of HPLC run: 12-15 min

The activity was calculated by calculating a µmol turnover after one minute, which corresponds to one U (unlit). Specific activities are given in U per g of DBM or mg of protein.

### Expressing the R. erythropolis 870-AN019 nitrile hydratase in a two-vector expression system

The constructs containing T7 promoters were expressed in accordance with the following protocol:
50 ml of LBₐₘₚ₁₀₀ medium containing 2 mM Fe citrate and in each case 50 µg of kanamycin and ampicillin/ml were inoculated to a concentration of 1% with an overnight culture. After an OD₆₀₀ of approx. 0.5 had been reached, expression of the nitrile hydratases was induced with 1 mM IPTG (isopropylthiogalactoside). The cells were harvested approx. 24 hours after inducing at 26°C.

### Expressing R. rhodochrous M8 nitrile hydratase in a two-vector expression system

The constructs containing T7 promoters were expressed in accordance with the following protocol:
50 ml of LBₐₘₚ₁₀₀ medium containing 0.5 mM CoCl₂ and in each case 50 µg of kanamycin and ampicillin/ml were inoculated to a concentration of 1% with an overnight culture. After an OD₆₀₀ of approx. 0.5 had been reached, expression of the NHases was induced with 1 mM IPTG (isopropylthiogalactoside). 3% (w/v) ethanol was also added to the medium. The cells were harvested approx. 24 hours after inducing at 26°C.

### Determining activity

The biotransformation for determining activity using the recombinant *R. rhodochrous* M8 and *R. erythropolis* 870-AN019 nitrile hydratases in *E. coli* took place on a small scale. The biotransformation is carried out in a 1.5 ml Eppendorf cup at 20°C. An OD of 0.4 was used in the biotransformation mixture. 500 µl of a 4% solution of acrylonitrile in 10 mM potassium phosphate buffer, pH 7.5, and also the buffer, are preincubated at 20°C. The reaction is started by adding the cells. The sum of the buffer volume and the cell volume is 500 µl. Immediately after the whole has been mixed, 100 µl are removed and pipetted into 1.5 µl of initially introduced concentrated HCl. After mixing, the sample is centrifuged down at 13 000 rpm for 2 min in an Eppendorf centrifuge and 70 µl of the supernatant are stored at -20°C for HPLC analysis. Samples were removed every 5 - 10 min over a period of at most 2 hours.

### Analyzing acrylonitrile, acrylamide and acrylic acid

Using the HPLC method which is described below, it is possible to rapidly analyze acrylonitrile, acrylamide and acrylic acid and to determine the concentrations of these substances:
Column: Synergi 4 µ Hydro-RP with precolumn
Mobile phase: 0.1% H₃PO₄ in 10% acetonitrile, 90% H₂O
Flow rate: 0.5 ml/min
Wavelength: 202 nm
Injection volume: 5 µl
Period of HPLC run: 10 min, last peak after 5.5 min

The activity was calculated by calculating a µmol turnover after one minute, which corresponds to one U (unit). Specific activities are given in U per g of DBM or mg of protein.

## Claims

1. An expression system for simultaneously expressing the nucleic acid sequences encoding the different subunits of a nitrile hydratase,
**characterized in that**
the expression system comprises different plasmids each containing at least one nucleic acid sequence encoding the respective subunit wherein the nucleic acid sequences encoding nitrile hydratase subunits are expressed separately on different plasmids.

2. The expression system as claimed in claim 1,
**characterized in that**
it is present in *E*.*coli* as host.

3. The expression system as claimed in one or both of the preceding claims,
**characterized in that**
the expression of the nucleic acid sequences encoding the subunits is under the control of what is in each case the same promoter.

4. The expression system as claimed in claim 3,
**characterized in that**
the promoter is a T7 promoter.

5. The expression system as claimed in one or more of the preceding claims,
**characterized in that**
at least one nucleic acid sequence encoding the p47K protein or the p12K protein is present per plasmid set employed.

6. The expression system as claimed in one or more of the preceding claims,
**characterized in that**
the nucleic acid sequences encoding the nitrile hydratase subunits are derived from rhodococcus strains.

7. The expression system as claimed in one or more of the preceding claims,
**characterized in that**
the nucleic acid sequences encoding the nitrile hydratase subunits are used in a form in which they are modified in accordance with the *E*.*coli* codon usage.

8. The expression system as claimed in one or more of the preceding claims,
**characterized in that**
the plasmids employed are those of the PET series.

9. A method for preparing nitrile hydratases using an expression system as claimed in one or more of claims 1 to 8.

10. A host organism which exhibits an expression system as claimed in one or more of claims 1 to 8.

11. A method for preparing (amino)carboxylic acids or (amino)carboxamides using a host organism as claimed in claim 10 or an expression system as claimed in one or more of claims 1 to 8.

12. A method as claimed in claim 11, wherein the (amino)carboxylic acids or (amino)carboxamides are enantiomerically enriched.

## Patentansprüche

1. Expressionssystem für die gleichzeitige Expression der die unterschiedlichen Untereinheiten einer Nitrilhydratase kodierenden Nukleinsäuresequenzen, **dadurch gekennzeichnet, dass** das Expressionssystem verschiedene Plasmide umfasst, von denen jedes mindestens eine Nukleinsäuresequenz enthält, die die jeweilige Untereinheit kodiert, wobei die Nitrilhydrataseuntereinheiten kodierenden Nukleinsäuresequenzen getrennt auf verschiedenen Plasmide exprimiert werden.

2. Expressionssystem nach Anspruch 1, **dadurch gekennzeichnet, dass** es in *E*. *coli* als Wirt vorhanden ist.

3. Expressionssystem nach einem der beiden vorstehenden Ansprüche, **dadurch gekennzeichnet, dass** die Expression der die Untereinheiten kodierenden Nukleinsäuresequenzen unter der Kontrolle des in beiden Fällen gleichen Pomotors steht.

4. Expressionssystem nach Anspruch 3, **dadurch gekennzeichnet, dass** der Promotor ein T7-Promotor ist.

5. Expressionssystem nach einem der vorstehenden Ansprüche, **dadurch gekennzeichnet, dass** mindestens eine Nukleinsäuresequenz, die das p47K-Protein oder das p12K-Protein kodiert, auf jedem eingesetzten Plasmidset vorhanden ist.

6. Expressionssystem nach einem der vorstehenden Ansprüche, **dadurch gekennzeichnet, dass** die die Nitrilhydrataseuntereinheiten kodierenden Nukleinsäuresequenzen von Rhodococcusstämmen abgeleitet sind.

7. Expressionssystem nach einem der vorstehenden Ansprüche, **dadurch gekennzeichnet, dass** die die Nitrilhydrataseuntereinheiten kodierenden Nukleinsäuresequenzen in einer Form eingesetzt werden, in der sie gemäß der Kodonverwendung von *E*. *coli* modifiziert sind.

8. Expressionssystem nach einem der vorstehenden Ansprüche, **dadurch gekennzeichnet, dass** die eingesetzten Plasmide zur pET-Serie gehören.

9. Verfahren zur Herstellung von Nitrilhydratasen, wobei ein Expressionssystem gemäß einem der Ansprüche 1 bis 8 verwendet wird.

10. Wirtsorganismus, der ein Expressionssystem nach einem der Ansprüche 1 bis 8 aufweist.

11. Verfahren zur Herstellung von Amino-Carbonsäuren oder Amino-Carboxamiden, wobei ein Wirtsorganismus nach Anspruch 10 oder ein Expressionssystem nach einem der Ansprüche 1 bis 8 eingesetzt wird.

12. Verfahren nach Anspruch 11, wobei die Amino-Carbonsäuren oder Amino-Carboxamide bezüglich der Enantiomere angereichert sind.

## Revendications

1. Système d'expression pour exprimer simultanément des séquences d'acide nucléique codant les différentes sous-unités d'une nitrile hydratase,
**caractérisé en ce que**
le système d'expression comprend des plasmides différents, chacun contenant au moins une séquence d'acide nucléique codant la sous-unité respective, les séquences d'acide nucléique codant les sous-unités de la nitrile hydratase étant exprimées séparément sur des plasmides différents.

2. Système d'expression tel que revendiqué dans la revendication 1,
**caractérisé en ce que**
il est présent dans un hôte *E*. *coli.*

3. Système d'expression tel que revendiqué dans l'une ou les deux revendications précédentes,
**caractérisé en ce que**
l'expression des séquences d'acide nucléique codant les sous-unités est sous contrôle de ce qui est dans chaque cas le même promoteur.

4. Système d'expression tel que revendiqué dans la revendication 3,
**caractérisé en ce que**
le promoteur est un promoteur T7.

5. Système d'expression tel que revendiqué dans une ou plusieurs des revendications précédentes,
**caractérisé en ce que**
au moins une séquence d'acide nucléique codant la protéine p47K ou la protéine p12K est présente par ensemble de plasmides utilisé.

6. Système d'expression tel que revendiqué dans une ou plusieurs des revendications précédentes,
**caractérisé en ce que**
les séquences d'acide nucléique codant les sous-unités de la nitrile hydratase sont dérivées de souches rhodococcus.

7. Système d'expression tel que revendiqué dans une ou plusieurs des revendications précédentes,
**caractérisé en ce que**
les séquences d'acide nucléique codant les sous-unités de la nitrile hydratase sont utilisées sous une forme modifiée selon l'usage des codons de *E*. *coli.*

8. Système d'expression tel que revendiqué dans une ou plusieurs des revendications précédentes,
**caractérisé en ce que**
les plasmides utilisés sont ceux de la série pET.

9. Méthode pour préparer des nitrile hydratases utilisant un système d'expression tel que revendiqué dans une ou plusieurs des revendications 1 à 8.

10. Organisme hôte qui présente un système d'expression tel que revendiqué dans une ou plusieurs des revendications 1 à 8.

11. Méthode pour préparer des acides (amino)carboxyliques ou des (amino)carboxamides utilisant un organisme hôte tel que revendiqué dans la revendication 10 ou un système d'expression tel que revendiqué dans une ou plusieurs des revendications 1 à 8.

12. Méthode telle que revendiquée dans la revendication 11, dans laquelle les acides (amino)carboxyliques ou les (amino)carboxamides sont enrichis en énantiomères.


### SEQUENCE LISTING

<110> Degussa AG
<120> Expression of nitrile hydratases in a two-vector expression system
<130> 040065 AM
<160> 34
<170> PatentIn version 3.1
<210> 1
   <211> 624
   <212> DNA
   <213> Rhodococcus erythropolis
<220>
   <221> CDS
   <222> (1)..(624)
   <223>
<400> 1
   atg tca gta acg atc gac cac aca acg gag aac gcc gca ccg gcc cag 48
   Met Ser Val Thr Ile Asp His Thr Thr Glu Asn Ala Ala Pro Ala Gln
   1 5 10 15
   gcg ccg gtc tcc gat cgc gcg tgg gcc ctg ttc cgc gca ctc gac ggt 96
   Ala Pro Val Ser Asp Arg Ala Trp Ala Leu Phe Arg Ala Leu Asp Gly
   20 25 30
   aag gga ttg gta ccc gac ggt tac gtc gag gga tgg aag aag acc ttc 144
   Lys Gly Leu Val Pro Asp Gly Tyr Val Glu Gly Trp Lys Lys Thr Phe
   35 40 45
   gag gag gac ttc agt cca agg cgc gga gcg gaa ttg gtc gcg cgg gcg 192
   Glu Glu Asp Phe Ser Pro Arg Arg Gly Ala Glu Leu Val Ala Arg Ala
   50 55 60
   tgg acc gac ccc gat ttc cgg caa ctg ctt ctc acc gac ggt acc gcc 240
   Trp Thr Asp Pro Asp Phe Arg Gln Leu Leu Leu Thr Asp Gly Thr Ala
   65 70 75 80
   gcg gtt gcc cag tac gga tat ctg ggc ccc cag ggc gaa tac atc gtg 288
   Ala Val Ala Gln Tyr Gly Tyr Leu Gly Pro Gln Gly Glu Tyr Ile Val
   85 90 95
   gca gtc gaa gac acc ccg acc ctc aag aac gtg atc gtg tgc tcg ctg 336
   Ala Val Glu Asp Thr Pro Thr Leu Lys Asn Val Ile Val Cys Ser Leu
   100 105 110
   tgt tca tgc acc gcg tgg ccc att ctc ggc ctg ccc cct acc tgg tac 384
   Cys Ser Cys Thr Ala Trp Pro Ile Leu Gly Leu Pro Pro Thr Trp Tyr
   115 120 125
   aag agt ttc gaa tac cgt gcg cga gtg gtg cgt gag cca cgg aag gtt 432
   Lys Ser Phe Glu Tyr Arg Ala Arg Val Val Arg Glu Pro Arg Lys Val
   130 135 140
   ctc ttc gag atg gga acc gag atc gcg tcg gac gtc gag atc cgc gtc 480
   Leu Phe Glu Met Gly Thr Glu Ile Ala Ser Asp Val Glu Ile Arg Val
   145 150 155 160
   tac gac acc acc gcc gaa act cgc tac atg gtt ctc ccg caa cgt ccc 528
   Tyr Asp Thr Thr Ala Glu Thr Arg Tyr Met Val Leu Pro Gln Arg Pro
   165 170 175
   gca ggc acc gaa ggc tgg agc cag gaa cag ctt cag gag atc gtc acc 576
   Ala Gly Thr Glu Gly Trp Ser Gln Glu Gln Leu Gln Glu Ile Val Thr
   180 185 190
   aag gac tgc ctg atc ggc gtc gca gtc ccg cag gtc ccc acc gtc tga 624
   Lys Asp Cys Leu Ile Gly Val Ala Val Pro Gln Val Pro Thr Val
   195 200 205
<210> 2
   <211> 207
   <212> PRT
   <213> Rhodococcus erythropolis
<400> 2
   Met Ser Val Thr Ile Asp His Thr Thr Glu Asn Ala Ala Pro Ala Gln
   1               5                   10                  15
   Ala Pro Val Ser Asp Arg Ala Trp Ala Leu Phe Arg Ala Leu Asp Gly
               20                  25                  30
   Lys Gly Leu Val Pro Asp Gly Tyr Val Glu Gly Trp Lys Lys Thr Phe
           35                  40                  45
   Glu Glu Asp Phe Ser Pro Arg Arg Gly Ala Glu Leu Val Ala Arg Ala
       50                  55                  60
   Trp Thr Asp Pro Asp Phe Arg Gln Leu Leu Leu Thr Asp Gly Thr Ala
   65                  70                  75                  80
   Ala Val Ala Gln Tyr Gly Tyr Leu Gly Pro Gln Gly Glu Tyr Ile Val
                   85                  90                  95
   Ala Val Glu Asp Thr Pro Thr Leu Lys Asn Val Ile Val Cys Ser Leu
               100                 105                 110
   Cys Ser Cys Thr Ala Trp Pro Ile Leu Gly Leu Pro Pro Thr Trp Tyr
           115                 120                 125
   Lys Ser Phe Glu Tyr Arg Ala Arg Val Val Arg Glu Pro Arg Lys Val
       130                 135                 140
   Leu Phe Glu Met Gly Thr Glu Ile Ala Ser Asp Val Glu Ile Arg Val
   145                 150                 155                 160
   Tyr Asp Thr Thr Ala Glu Thr Arg Tyr Met Val Leu Pro Gln Arg Pro
                   165                 170                 175
   Ala Gly Thr Glu Gly Trp Ser Gln Glu Gln Leu Gln Glu Ile Val Thr
               180                 185                 190
   Lys Asp Cys Leu Ile Gly Val Ala Val Pro Gln Val Pro Thr Val
           195                 200                 205
<210> 3
   <211> 639
   <212> DNA
   <213> Rhodococcus erythropolis
<220>
   <221> CDS
   <222> (1)..(639)
   <223>
<400> 3
   atg gat gga gta cac gat ctt gcc gga gtt caa ggc ttc ggc aaa gtc      48
   Met Asp Gly Val His Asp Leu Ala Gly Val Gln Gly Phe Gly Lys Val
   1               5                   10                  15
   ccg cat acc gtc aac gcc gac atc ggc ccc acc ttc cac gcc gag tgg      96
   Pro His Thr Val Asn Ala Asp Ile Gly Pro Thr Phe His Ala Glu Trp
               20                  25                  30
   gaa cac ctg ccg tac agc ctg atg ttc gcc ggt gtc gcc gaa ctc ggg     144
   Glu His Leu Pro Tyr Ser Leu Met Phe Ala Gly Val Ala Glu Leu Gly
           35                  40                  45
   gca ttc agc gtc gac gaa gtt cga tac gtc gtc gag cgg atg gaa cca     192
   Ala Phe Ser Val Asp Glu Val Arg Tyr Val Val Glu Arg Met Glu Pro
       50                  55                  60
   cgc cac tac atg atg acc ccg tac tac gag agg tac gtc atc ggc gtc     240
   Arg His Tyr Met Met Thr Pro Tyr Tyr Glu Arg Tyr Val Ile Gly Val
   65                  70                  75                  80
   gcg aca ctg atg gtc gaa aag gga atc ctg acg cag gaa gaa ctc gaa     288
   Ala Thr Leu Met Val Glu Lys Gly Ile Leu Thr Gln Glu Glu Leu Glu
                   85                  90                  95
   agc ctt gca ggg gga ccg ttc cca ctg tcg cgg ccc agc gaa tcc gaa     336
   Ser Leu Ala Gly Gly Pro Phe Pro Leu Ser Arg Pro Ser Glu Ser Glu
               100                 105                 110
   ggg cgg ccg gca ccc gtc gag acg acc acc ttc gaa atc ggt cag cga     384
   Gly Arg Pro Ala Pro Val Glu Thr Thr Thr Phe Glu Ile Gly Gln Arg
           115                 120                 125
   gta cgc gtg cgc gac gag tac gtt ccg ggg cat att cga atg cct gcg       432
   Val Arg Val Arg Asp Glu Tyr Val Pro Gly His Ile Arg Met Pro Ala
       130                 135                 140
   tac tgc cgc gga cga gtg gga acc atc tct cat cgg act acc gag aag       480
   Tyr Cys Arg Gly Arg Val Gly Thr Ile Ser His Arg Thr Thr Glu Lys
   145                 150                 155                 160
   tgg ccg ttt ccc gac gca atc ggc cac ggg cgc aac gac gcc ggc gaa       528
   Trp Pro Phe Pro Asp Ala Ile Gly His Gly Arg Asn Asp Ala Gly Glu
                   165                 170                 175
   gaa ccg acg tac cac gtg aag ttc gac gcc gag gaa ttg ttc ggt agc       576
   Glu Pro Thr Tyr His Val Lys Phe Asp Ala Glu Glu Leu Phe Gly Ser
               180                 185                 190
   gac acc gac ggc ggc agc gtc gta gtc gac ctt ttc gag ggt tac ctc       624
   Asp Thr Asp Gly Gly Ser Val Val Val Asp Leu Phe Glu Gly Tyr Leu
           195                 200                 205
   gag cct gcg gcc tga                                                   639
   Glu Pro Ala Ala
       210
<210> 4
   <211> 212
   <212> PRT
   <213> Rhodococcus erythropolis
<400> 4
   Met Asp Gly Val His Asp Leu Ala Gly Val Gln Gly Phe Gly Lys Val
   1 5 10 15
   Pro His Thr Val Asn Ala Asp Ile Gly Pro Thr Phe His Ala Glu Trp
   20 25 30
   Glu His Leu Pro Tyr Ser Leu Met Phe Ala Gly Val Ala Glu Leu Gly
   35 40 45
   Ala Phe Ser Val Asp Glu Val Arg Tyr Val Val Glu Arg Met Glu Pro
   50 55 60
   Arg His Tyr Met Met Thr Pro Tyr Tyr Glu Arg Tyr Val Ile Gly Val
   65 70 75 80
   Ala Thr Leu Met Val Glu Lys Gly Ile Leu Thr Gln Glu Glu Leu Glu
   85 90 95
   Ser Leu Ala Gly Gly Pro Phe Pro Leu Ser Arg Pro Ser Glu Ser Glu
   100 105 110
   Gly Arg Pro Ala Pro Val Glu Thr Thr Thr Phe Glu Ile Gly Gln Arg
   115 120 125
   Val Arg Val Arg Asp Glu Tyr Val Pro Gly His Ile Arg Met Pro Ala
   130 135 140
   Tyr Cys Arg Gly Arg Val Gly Thr Ile Ser His Arg Thr Thr Glu Lys
   145 150 155 160
   Trp Pro Phe Pro Asp Ala Ile Gly His Gly Arg Asn Asp Ala Gly Glu
   165 170 175
   Glu Pro Thr Tyr His Val Lys Phe Asp Ala Glu Glu Leu Phe Gly Ser
   180 185 190
   Asp Thr Asp Gly Gly Ser Val Val Val Asp Leu Phe Glu Gly Tyr Leu
   195 200 205
   Glu Pro Ala Ala
   210
<210> 5
   <211> 624
   <212> DNA
   <213> Rhodococcus erythropolis
<220>
   <221> CDS
   <222> (1)..(624)
   <223>
<400> 5
   atg tca gta acg atc gac cac aca acg gag aac gcc gca ccg gcc cag        48
   Met Ser Val Thr Ile Asp His Thr Thr Glu Asn Ala Ala Pro Ala Gln
   1               5                   10                  15
   gcg ccg gtc tcc gat cgc gcg tgg gcc ctg ttc cgc gca ctc gac ggt        96
   Ala Pro Val Ser Asp Arg Ala Trp Ala Leu Phe Arg Ala Leu Asp Gly
               20                  25                  30
   aag gga ttg gta ccc gac ggt tac gtc gaa gga tgg aag aaa acc ttc       144
   Lys Gly Leu Val Pro Asp Gly Tyr Val Glu Gly Trp Lys Lys Thr Phe
           35                  40                  45
   gag gag gac ttc agt cca agg cgc gga gcg gaa ttg gtc gcg cgg gcg       192
   Glu Glu Asp Phe Ser Pro Arg Arg Gly Ala Glu Leu Val Ala Arg Ala
       50                  55                  60
   tgg acc gac ccc gag ttc cgg cag ttg ctt ctc acc gac ggt acc gcc       240
   Trp Thr Asp Pro Glu Phe Arg Gln Leu Leu Leu Thr Asp Gly Thr Ala
   65                  70                  75                  80
   gcg gtt gcc cag tac gga tac ctg ggc ccc cag ggc gag tac atc gtg       288
   Ala Val Ala Gln Tyr Gly Tyr Leu Gly Pro Gln Gly Glu Tyr Ile Val
                   85                  90                  95
   gca gtc gaa gac acc ccg acc ctc aag aac gtg atc gtg tgc tcg ctg       336
   Ala Val Glu Asp Thr Pro Thr Leu Lys Asn Val Ile Val Cys Ser Leu
               100                 105                 110
   tgt tca tgc acc gcg tgg ccc att ctc ggc ctg ccc cct acc tgg tac       384
   Cys Ser Cys Thr Ala Trp Pro Ile Leu Gly Leu Pro Pro Thr Trp Tyr
           115                 120                 125
   aag agt ttc gaa tac cgt gcg cga gtg gtg cgt gag cca cgg aag gtt       432
   Lys Ser Phe Glu Tyr Arg Ala Arg Val Val Arg Glu Pro Arg Lys Val
       130                 135                 140
   ctc tcc gag atg gga acc gag atc gcg tcg gac gtc gag atc cgc gtc       480
   Leu Ser Glu Met Gly Thr Glu Ile Ala Ser Asp Val Glu Ile Arg Val
   145                 150                 155                 160
   tac gac acc acc gcc gaa act cgc tac atg gtt ctc ccg caa cgt ccc       528
   Tyr Asp Thr Thr Ala Glu Thr Arg Tyr Met Val Leu Pro Gln Arg Pro
                   165                 170                 175
   gca ggc acc gaa ggc tgg agc cag gaa caa ctg cag gaa atc gtc acc       576
   Ala Gly Thr Glu Gly Trp Ser Gln Glu Gln Leu Gln Glu Ile Val Thr
               180                 185                 190
   aag gac tgc ctg atc ggc gtc gca gtc ccg cag gtc ccc acc gtc tga       624
   Lys Asp Cys Leu Ile Gly Val Ala Val Pro Gln Val Pro Thr Val
           195                 200                 205
<210> 6
   <211> 207
   <212> PRT
   <213> Rhodococcus erythropolis
<400> 6
   Met Ser Val Thr Ile Asp His Thr Thr Glu Asn Ala Ala Pro Ala Gln
   1               5                   10                  15
   Ala Pro Val Ser Asp Arg Ala Trp Ala Leu Phe Arg Ala Leu Asp Gly
               20                  25                  30
   Lys Gly Leu Val Pro Asp Gly Tyr Val Glu Gly Trp Lys Lys Thr Phe
           35                  40                  45
   Glu Glu Asp Phe Ser Pro Arg Arg Gly Ala Glu Leu Val Ala Arg Ala
       50                  55                  60
   Trp Thr Asp Pro Glu Phe Arg Gln Leu Leu Leu Thr Asp Gly Thr Ala
   65 70 75 80
   Ala Val Ala Gln Tyr Gly Tyr Leu Gly Pro Gln Gly Glu Tyr Ile Val
   85 90 95
   Ala Val Glu Asp Thr Pro Thr Leu Lys Asn Val Ile Val Cys Ser Leu
   100 105 110
   Cys Ser Cys Thr Ala Trp Pro Ile Leu Gly Leu Pro Pro Thr Trp Tyr
   115 120 125
   Lys Ser Phe Glu Tyr Arg Ala Arg Val Val Arg Glu Pro Arg Lys Val
   130 135 140
   Leu Ser Glu Met Gly Thr Glu Ile Ala Ser Asp Val Glu Ile Arg Val
   145 150 155 160
   Tyr Asp Thr Thr Ala Glu Thr Arg Tyr Met Val Leu Pro Gln Arg Pro
   165 170 175
   Ala Gly Thr Glu Gly Trp Ser Gln Glu Gln Leu Gln Glu Ile Val Thr
   180 185 190
   Lys Asp Cys Leu Ile Gly Val Ala Val Pro Gln Val Pro Thr Val
   195 200 205
<210> 7
   <211> 639
   <212> DNA
   <213> Rhodococcus erythropolis
<220>
   <221> CDS
   <222> (1)..(639)
   <223>
<400> 7
   atg gat gga gta cac gat ctt gcc gga gtt caa ggc ttc ggc aaa gtc        48
   Met Asp Gly Val His Asp Leu Ala Gly Val Gln Gly Phe Gly Lys Val
   1               5                   10                  15
   ccg cat acc gtc aac gcc gac atc ggc ccc acc ttc cac gcc gag tgg        96
   Pro His Thr Val Asn Ala Asp Ile Gly Pro Thr Phe His Ala Glu Trp
               20                  25                  30
   gaa cac ctg ccg tac agc ctg atg ttc gcc ggt gtc gcc gaa ctc ggg       144
   Glu His Leu Pro Tyr Ser Leu Met Phe Ala Gly Val Ala Glu Leu Gly
           35                  40                  45
   gca ttc agc gtc gac gaa gtt cga tac gtc gtc gag cgg atg gaa cca       192
   Ala Phe Ser Val Asp Glu Val Arg Tyr Val Val Glu Arg Met Glu Pro
       50                  55                  60
   cgc cac tac atg atg acc ccg tac tac gag agg tac gtc atc ggc gtc       240
   Arg His Tyr Met Met Thr Pro Tyr Tyr Glu Arg Tyr Val Ile Gly Val
   65                  70                  75                  80
   gcg aca ctg atg gtc gaa aag gga atc ctg acg cag gat gaa ctc gaa       288
   Ala Thr Leu Met Val Glu Lys Gly Ile Leu Thr Gln Asp Glu Leu Glu
                   85                  90                  95
   agc ctt gca ggg gga ccg ttc cca ctg tcg cgg ccc agc gaa tcc gaa       336
   Ser Leu Ala Gly Gly Pro Phe Pro Leu Ser Arg Pro Ser Glu Ser Glu
               100                 105                 110
   ggg cgt ccg gca ccc gtc gag acg acc acc ttc gaa atc ggt cag cga       384
   Gly Arg Pro Ala Pro Val Glu Thr Thr Thr Phe Glu Ile Gly Gln Arg
           115                 120                 125
   gta cgc gtg cgc gac gag tac gtt ccg ggg cat att cga atg cct gcg       432
   Val Arg Val Arg Asp Glu Tyr Val Pro Gly His Ile Arg Met Pro Ala
       130                 135                 140
   tac tgc cgc gga cga gtg gga acc atc tct cat cgg act acc gag aag       480
   Tyr Cys Arg Gly Arg Val Gly Thr Ile Ser His Arg Thr Thr Glu Lys
   145                 150                 155                 160
   tgg cca ttt ccc gac gca atc ggc cac ggg cgc aac gac gcc ggc gaa       528
   Trp Pro Phe Pro Asp Ala Ile Gly His Gly Arg Asn Asp Ala Gly Glu
                   165                 170                 175
   gaa ccg acg tac cac gtg aag ttc gcc gcc gag gaa ttg ttc ggt agc       576
   Glu Pro Thr Tyr His Val Lys Phe Ala Ala Glu Glu Leu Phe Gly Ser
               180                 185                 190
   gac acc gac ggc ggc agc gtc gta gtc gac ctt ttc gag ggt tac ctc       624
   Asp Thr Asp Gly Gly Ser Val Val Val Asp Leu Phe Glu Gly Tyr Leu
           195                 200                 205
   gag cct gcg gcc tga                                                   639
   Glu Pro Ala Ala
       210
<210> 8
   <211> 212
   <212> PRT
   <213> Rhodococcus erythropolis
<400> 8
   Met Asp Gly Val His Asp Leu Ala Gly Val Gln Gly Phe Gly Lys Val
   1 5 10 15
   Pro His Thr Val Asn Ala Asp Ile Gly Pro Thr Phe His Ala Glu Trp
   20 25 30
   Glu His Leu Pro Tyr Ser Leu Met Phe Ala Gly Val Ala Glu Leu Gly
   35 40 45
   Ala Phe Ser Val Asp Glu Val Arg Tyr Val Val Glu Arg Met Glu Pro
   50 55 60
   Arg His Tyr Met Met Thr Pro Tyr Tyr Glu Arg Tyr Val Ile Gly Val
   65 70 75 80
   Ala Thr Leu Met Val Glu Lys Gly Ile Leu Thr Gln Asp Glu Leu Glu
   85 90 95
   Ser Leu Ala Gly Gly Pro Phe Pro Leu Ser Arg Pro Ser Glu Ser Glu
   100 105 110
   Gly Arg Pro Ala Pro Val Glu Thr Thr Thr Phe Glu Ile Gly Gln Arg
   115 120 125
   Val Arg Val Arg Asp Glu Tyr Val Pro Gly His Ile Arg Met Pro Ala
   130 135 140
   Tyr Cys Arg Gly Arg Val Gly Thr Ile Ser His Arg Thr Thr Glu Lys
   145 150 155 160
   Trp Pro Phe Pro Asp Ala Ile Gly His Gly Arg Asn Asp Ala Gly Glu
   165 170 175
   Glu Pro Thr Tyr His Val Lys Phe Ala Ala Glu Glu Leu Phe Gly Ser
   180 185 190
   Asp Thr Asp Gly Gly Ser Val Val Val Asp Leu Phe Glu Gly Tyr Leu
   195 200 205
   Glu Pro Ala Ala
   210
<210> 9
   <211> 624
   <212> DNA
   <213> Rhodococcus erythropolis
<220>
   <221> CDS
   <222> (1)..(624)
   <223>
<400> 9
   atg tca gta acg atc gac cac aca acg gag aac gcc gca ccg gcc cag      48
   Met Ser Val Thr Ile Asp His Thr Thr Glu Asn Ala Ala Pro Ala Gln
   1               5                   10                  15
   gcg ccg gtc tcc gac cgg gcg tgg gcc ctg ttc cgc gca ctc gac ggt      96
   Ala Pro Val Ser Asp Arg Ala Trp Ala Leu Phe Arg Ala Leu Asp Gly
               20                  25                  30
   aag gga ttg gta ccc gac ggt tac gtc gag gga tgg aag aag acc ttc     144
   Lys Gly Leu Val Pro Asp Gly Tyr Val Glu Gly Trp Lys Lys Thr Phe
           35                  40                  45
   gag gag gac ttc agt cca agg cgc gga gcg gaa ttg gtc gcg cgg gcg     192
   Glu Glu Asp Phe Ser Pro Arg Arg Gly Ala Glu Leu Val Ala Arg Ala
       50                  55                  60
   tgg acc gac ccc gag ttc cgg cag ttg ctt ctc acc gac ggt acc gcc     240
   Trp Thr Asp Pro Glu Phe Arg Gln Leu Leu Leu Thr Asp Gly Thr Ala
   65                  70                  75                  80
   gcg gtt gcc cag tac gga tat ctg ggc ccc cag ggc gag tac atc gtg     288
   Ala Val Ala Gln Tyr Gly Tyr Leu Gly Pro Gln Gly Glu Tyr Ile Val
                   85                  90                  95
   gca gtc gaa gac acc ccg acc ctc aag aac gtg atc gtg tgc tcg ttg     336
   Ala Val Glu Asp Thr Pro Thr Leu Lys Asn Val Ile Val Cys Ser Leu
               100                 105                 110
   tgt tca tgc acc gcg tgg ccc att ctc ggc ctg ccc cct acc tgg tac     384
   Cys Ser Cys Thr Ala Trp Pro Ile Leu Gly Leu Pro Pro Thr Trp Tyr
           115                 120                 125
   aag agt ttc gaa tac cgt gcg cga gtg gtg cgt gag cca cgg aag gtt     432
   Lys Ser Phe Glu Tyr Arg Ala Arg Val Val Arg Glu Pro Arg Lys Val
       130                 135                 140
   ctc tcc gag atg gga acc gag atc gcg tcg gac gtc gag atc cgc gtc     480
   Leu Ser Glu Met Gly Thr Glu Ile Ala Ser Asp Val Glu Ile Arg Val
   145                 150                 155                 160
   tac gac acc acc gcc gaa act cgc tac atg gtt ctc ccg caa cgt ccc     528
   Tyr Asp Thr Thr Ala Glu Thr Arg Tyr Met Val Leu Pro Gln Arg Pro
                   165                 170                 175
   gca ggc acc gaa ggc tgg agc cag gaa cag ctt caa gag atc gtc acc     576
   Ala Gly Thr Glu Gly Trp Ser Gln Glu Gln Leu Gln Glu Ile Val Thr
               180                 185                 190
   aag gac tgc ctg atc ggc gtc gca gtc ccg cag gtc ccc acc gtc tga     624
   Lys Asp Cys Leu Ile Gly Val Ala Val Pro Gln Val Pro Thr Val
           195                 200                 205
<210> 10
   <211> 207
   <212> PRT
   <213> Rhodococcus erythropolis
<400> 10
   Met Ser Val Thr Ile Asp His Thr Thr Glu Asn Ala Ala Pro Ala Gln
   1 5 10 15
   Ala Pro Val Ser Asp Arg Ala Trp Ala Leu Phe Arg Ala Leu Asp Gly
   20 25 30
   Lys Gly Leu Val Pro Asp Gly Tyr Val Glu Gly Trp Lys Lys Thr Phe
   35 40 45
   Glu Glu Asp Phe Ser Pro Arg Arg Gly Ala Glu Leu Val Ala Arg Ala
   50 55 60
   Trp Thr Asp Pro Glu Phe Arg Gln Leu Leu Leu Thr Asp Gly Thr Ala
   65 70 75 80
   Ala Val Ala Gln Tyr Gly Tyr Leu Gly Pro Gln Gly Glu Tyr Ile Val
   85 90 95
   Ala Val Glu Asp Thr Pro Thr Leu Lys Asn Val Ile Val Cys Ser Leu
   100 105 110
   Cys Ser Cys Thr Ala Trp Pro Ile Leu Gly Leu Pro Pro Thr Trp Tyr
   115 120 125
   Lys Ser Phe Glu Tyr Arg Ala Arg Val Val Arg Glu Pro Arg Lys Val
   130 135 140
   Leu Ser Glu Met Gly Thr Glu Ile Ala Ser Asp Val Glu Ile Arg Val
   145 150 155 160
   Tyr Asp Thr Thr Ala Glu Thr Arg Tyr Met Val Leu Pro Gln Arg Pro
   165 170 175
   Ala Gly Thr Glu Gly Trp Ser Gln Glu Gln Leu Gln Glu Ile Val Thr
   180 185 190
   Lys Asp Cys Leu Ile Gly Val Ala Val Pro Gln Val Pro Thr Val
   195 200 205
<210> 11
   <211> 639
   <212> DNA
   <213> Rhodococcus erythropolis
<220>
   <221> CDS
   <222> (1)..(639)
   <223>
<400> 11
   atg gat gga gta cac gat ctt gcc gga gtt caa ggc ttc ggc aaa gtc      48
   Met Asp Gly Val His Asp Leu Ala Gly Val Gln Gly Phe Gly Lys Val
   1               5                   10                  15
   ccg cat acc gtc aac gcc gac atc ggc ccc acc ttc cac gcc gag tgg      96
   Pro His Thr Val Asn Ala Asp Ile Gly Pro Thr Phe His Ala Glu Trp
               20                  25                  30
   gaa cac ctg ccg tac agc ctg atg ttc gcc ggt gtc gcc gaa ctc ggg     144
   Glu His Leu Pro Tyr Ser Leu Met Phe Ala Gly Val Ala Glu Leu Gly
           35                  40                  45
   gca ttc agc gtc gac gaa gtt cga tac gtc gtc gag cgg atg gaa cca     192
   Ala Phe Ser Val Asp Glu Val Arg Tyr Val Val Glu Arg Met Glu Pro
       50                  55                  60
   cgc cac tac atg atg acc ccg tac tac gag agg tac gtc atc ggc gtc     240
   Arg His Tyr Met Met Thr Pro Tyr Tyr Glu Arg Tyr Val Ile Gly Val
   65                  70                  75                  80
   gcg aca ctg atg gtc gaa aag gga atc ctg acg cag gaa gaa ctc gaa     288
   Ala Thr Leu Met Val Glu Lys Gly Ile Leu Thr Gln Glu Glu Leu Glu
                   85                  90                  95
   agc ctt gca ggg gga ccg ttc cca ctg tcg cgg cca agc gaa tcc gaa     336
   Ser Leu Ala Gly Gly Pro Phe Pro Leu Ser Arg Pro Ser Glu Ser Glu
               100                 105                 110
   ggg cgt ccg gca ccc gtc gag acg acc acc ttc gaa gtc ggt cag cga     384
   Gly Arg Pro Ala Pro Val Glu Thr Thr Thr Phe Glu Val Gly Gln Arg
           115                 120                 125
   gta cgc gtg cgc gac gag tac gtt ccg ggg cat att cga atg cct gcg     432
   Val Arg Val Arg Asp Glu Tyr Val Pro Gly His Ile Arg Met Pro Ala
       130                 135                 140
   tac tgc cgc gga cga gtg gga acc atc tct cat cgg act acc gag aag     480
   Tyr Cys Arg Gly Arg Val Gly Thr Ile Ser His Arg Thr Thr Glu Lys
   145                 150                 155                 160
   tgg cca ttt ccc gac gca atc ggc cac ggg cgc aac gac gcc ggc gaa     528
   Trp Pro Phe Pro Asp Ala Ile Gly His Gly Arg Asn Asp Ala Gly Glu
                   165                 170                 175
   gaa ccg acg tac cac gtg aag ttc gac gcc gag gaa ttg ttc ggt agc     576
   Glu Pro Thr Tyr His Val Lys Phe Asp Ala Glu Glu Leu Phe Gly Ser
               180                 185                 190
   gac acc gac ggc ggc agc gtc gta gtc gac ctt ttc gag ggt tac ctc     624
   Asp Thr Asp Gly Gly Ser Val Val Val Asp Leu Phe Glu Gly Tyr Leu
           195                 200                 205
   gag cct gcg gcc tga                                                    639
   Glu Pro Ala Ala
       210
<210> 12
   <211> 212
   <212> PRT
   <213> Rhodococcus erythropolis
<400> 12
   Met Asp Gly Val His Asp Leu Ala Gly Val Gln Gly Phe Gly Lys Val
   1               5                   10                  15
   Pro His Thr Val Asn Ala Asp Ile Gly Pro Thr Phe His Ala Glu Trp
               20                  25                  30
   Glu His Leu Pro Tyr Ser Leu Met Phe Ala Gly Val Ala Glu Leu Gly
           35                  40                  45
   Ala Phe Ser Val Asp Glu Val Arg Tyr Val Val Glu Arg Met Glu Pro
       50                  55                  60
   Arg His Tyr Met Met Thr Pro Tyr Tyr Glu Arg Tyr Val Ile Gly Val
   65                  70                  75                  80
   Ala Thr Leu Met Val Glu Lys Gly Ile Leu Thr Gln Glu Glu Leu Glu
                   85                  90                  95
   Ser Leu Ala Gly Gly Pro Phe Pro Leu Ser Arg Pro Ser Glu Ser Glu
               100                 105                 110
   Gly Arg Pro Ala Pro Val Glu Thr Thr Thr Phe Glu Val Gly Gln Arg
           115                 120                 125
   Val Arg Val Arg Asp Glu Tyr Val Pro Gly His Ile Arg Met Pro Ala
       130                 135                 140
   Tyr Cys Arg Gly Arg Val Gly Thr Ile Ser His Arg Thr Thr Glu Lys
   145                 150                 155                 160
   Trp Pro Phe Pro Asp Ala Ile Gly His Gly Arg Asn Asp Ala Gly Glu
                   165                 170                 175
   Glu Pro Thr Tyr His Val Lys Phe Asp Ala Glu Glu Leu Phe Gly Ser
               180                 185                 190
   Asp Thr Asp Gly Gly Ser Val Val Val Asp Leu Phe Glu Gly Tyr Leu
           195                 200                 205
   Glu Pro Ala Ala
       210
<210> 13
   <211> 612
   <212> DNA
   <213> Rhodococcus erythropolis
<220>
   <221> CDS
   <222> (1)..(612)
   <223>
<400> 13
   gtg agc gag cac gtc aat aag tac acg gag tac gag gca cgt acc aag        48
   Val Ser Glu His Val Asn Lys Tyr Thr Glu Tyr Glu Ala Arg Thr Lys
   1               5                   10                  15
   gca atc gaa act ttg ctg tac gag cga ggg ctc atc acg ccc gcc gcg        96
   Ala Ile Glu Thr Leu Leu Tyr Glu Arg Gly Leu Ile Thr Pro Ala Ala
               20                  25                  30
   gtc gac cga gtc gtt tcg tac tac gag aac gag atc ggc ccg atg ggc       144
   Val Asp Arg Val Val Ser Tyr Tyr Glu Asn Glu Ile Gly Pro Met Gly
           35                  40                  45
   ggt gcc aag gtc gtg gcg aag tcc tgg gtg gac cct gag tac cgc aag       192
   Gly Ala Lys Val Val Ala Lys Ser Trp Val Asp Pro Glu Tyr Arg Lys
       50                  55                  60
   tgg ctc gaa gag gac gcg acg gcc gcg atg gcg tca ttg ggc tat gcc       240
   Trp Leu Glu Glu Asp Ala Thr Ala Ala Met Ala Ser Leu Gly Tyr Ala
   65                  70                  75                  80
   ggt gag cag gca cac caa att tcg gcg gtc ttc aac gac tcc caa acg       288
   Gly Glu Gln Ala His Gln Ile Ser Ala Val Phe Asn Asp Ser Gln Thr
                   85                  90                  95
   cat cac gtg gtg gtg tgc act ctg tgt tcg tgc tat ccg tgg ccg gtg       336
   His His Val Val Val Cys Thr Leu Cys Ser Cys Tyr Pro Trp Pro Val
               100                 105                 110
   ctt ggt ctc ccg ccc gcc tgg tac aag agc atg gag tac cgg tcc cga       384
   Leu Gly Leu Pro Pro Ala Trp Tyr Lys Ser Met Glu Tyr Arg Ser Arg
           115                 120                 125
   gtg gta gcg gac cct cgt gga gtg ctc aag cgc gat ttc ggt ttc gac       432
   Val Val Ala Asp Pro Arg Gly Val Leu Lys Arg Asp Phe Gly Phe Asp
       130                 135                 140
   atc ccc gat gag gtg gag gtc agg gtt tgg gac agc agc tcc gaa atc       480
   Ile Pro Asp Glu Val Glu Val Arg Val Trp Asp Ser Ser Ser Glu Ile
   145                 150                 155                 160
   cgc tac atc gtc atc ccg gaa cgg ccg gcc ggc acc gac ggt tgg tcc         528
   Arg Tyr Ile Val Ile Pro Glu Arg Pro Ala Gly Thr Asp Gly Trp Ser
                   165                 170                 175
   gag gac gag ctg gcg aag ctg gtg agt cgg gac tcg atg atc ggt gtc         576
   Glu Asp Glu Leu Ala Lys Leu Val Ser Arg Asp Ser Met Ile Gly Val
               180                 185                 190
   agt aat gcg ctc aca ccc cag gaa gtg atc gta tga                         612
   Ser Asn Ala Leu Thr Pro Gln Glu Val Ile Val
           195                 200
<210> 14
   <211> 203
   <212> PRT
   <213> Rhodococcus erythropolis
<400> 14
   Val Ser Glu His Val Asn Lys Tyr Thr Glu Tyr Glu Ala Arg Thr Lys
   1               5                   10                  15
   Ala Ile Glu Thr Leu Leu Tyr Glu Arg Gly Leu Ile Thr Pro Ala Ala
               20                  25                  30
   Val Asp Arg Val Val Ser Tyr Tyr Glu Asn Glu Ile Gly Pro Met Gly
           35                  40                  45
   Gly Ala Lys Val Val Ala Lys Ser Trp Val Asp Pro Glu Tyr Arg Lys
       50                  55                  60
   Trp Leu Glu Glu Asp Ala Thr Ala Ala Met Ala Ser Leu Gly Tyr Ala
   65                  70                  75                  80
   Gly Glu Gln Ala His Gln Ile Ser Ala Val Phe Asn Asp Ser Gln Thr
                   85                  90                  95
   His His Val Val Val Cys Thr Leu Cys Ser Cys Tyr Pro Trp Pro Val
               100                 105                 110
   Leu Gly Leu Pro Pro Ala Trp Tyr Lys Ser Met Glu Tyr Arg Ser Arg
           115                 120                 125
   Val Val Ala Asp Pro Arg Gly Val Leu Lys Arg Asp Phe Gly Phe Asp
       130                 135                 140
   Ile Pro Asp Glu Val Glu Val Arg Val Trp Asp Ser Ser Ser Glu Ile
   145                 150                 155                 160
   Arg Tyr Ile Val Ile Pro Glu Arg Pro Ala Gly Thr Asp Gly Trp Ser
                   165                 170                 175
   Glu Asp Glu Leu Ala Lys Leu Val Ser Arg Asp Ser Met Ile Gly Val
               180                 185                 190
   Ser Asn Ala Leu Thr Pro Gln Glu Val Ile Val
           195                 200
<210> 15
   <211> 690
   <212> DNA
   <213> Rhodococcus erythropolis
<220>
   <221> CDS
   <222> (1)..(690)
   <223>
<400> 15
   atg gat ggt atc cac gac aca ggc ggc atg acc gga tac gga ccg gtc       48
   Met Asp Gly Ile His Asp Thr Gly Gly Met Thr Gly Tyr Gly Pro Val
   1               5                   10                  15
   ccc tat cag aag gac gag ccc ttc ttc cac tac gag tgg gag ggt cgg       96
   Pro Tyr Gln Lys Asp Glu Pro Phe Phe His Tyr Glu Trp Glu Gly Arg
               20                  25                  30
   acc ctg tcg att ctg acc tgg atg cat ctc aag ggc atg tcg tgg tgg      144
   Thr Leu Ser Ile Leu Thr Trp Met His Leu Lys Gly Met Ser Trp Trp
           35                  40                  45
   gac aag tcg cgg ttc ttc cgg gag tcg atg ggg aac gaa aac tac gtc      192
   Asp Lys Ser Arg Phe Phe Arg Glu Ser Met Gly Asn Glu Asn Tyr Val
       50                  55                  60
   aac gag att cgc aac tcg tac tac acc cac tgg ctg agt gcg gca gaa      240
   Asn Glu Ile Arg Asn Ser Tyr Tyr Thr His Trp Leu Ser Ala Ala Glu
   65                  70                  75                  80
   cgt atc ctc gtc gcc gac aag atc atc acc gaa gaa gag cga aag cac      288
   Arg Ile Leu Val Ala Asp Lys Ile Ile Thr Glu Glu Glu Arg Lys His
                   85                  90                  95
   cgt gtg cag gag atc ctc gag ggt cgg tac acg gac agg aac ccg tcg      336
   Arg Val Gln Glu Ile Leu Glu Gly Arg Tyr Thr Asp Arg Asn Pro Ser
               100                 105                 110
   cgg aag ttc gat ccg gcc gag atc gag aag gcg atc gaa cgg ctt cac      384
   Arg Lys Phe Asp Pro Ala Glu Ile Glu Lys Ala Ile Glu Arg Leu His
           115                 120                 125
   gag ccc cac tcc cta gca ctt cca gga gcg gag ccg agt ttc tcc ctc      432
   Glu Pro His Ser Leu Ala Leu Pro Gly Ala Glu Pro Ser Phe Ser Leu
       130                 135                 140
   ggt gac aag gtc aaa gtg aag aat atg aac ccg ctg gga cac aca cgg      480
   Gly Asp Lys Val Lys Val Lys Asn Met Asn Pro Leu Gly His Thr Arg
   145                 150                 155                 160
   tgc ccg aaa tat gtg cgg aac aag atc ggg gaa atc gtc acc tcc cac      528
   Cys Pro Lys Tyr Val Arg Asn Lys Ile Gly Glu Ile Val Thr Ser His
                   165                 170                 175
   ggc tgc cag atc tat ccc gag agc agc tcc gcc ggc ctc ggc gac gat      576
   Gly Cys Gln Ile Tyr Pro Glu Ser Ser Ser Ala Gly Leu Gly Asp Asp
               180                 185                 190
   ccc cgc ccg ctc tac acg gtc gcg ttt tcc gcc cag gaa ctg tgg ggc      624
   Pro Arg Pro Leu Tyr Thr Val Ala Phe Ser Ala Gln Glu Leu Trp Gly
           195                 200                 205
   gac gac gga aac ggg aaa gac gta gtg tgc gtc gat ctc tgg gaa ccg      672
   Asp Asp Gly Asn Gly Lys Asp Val Val Cys Val Asp Leu Trp Glu Pro
       210                 215                 220
   tac ctg atc tct gcg tga                                              690
   Tyr Leu Ile Ser Ala
   225
<210> 16
   <211> 229
   <212> PRT
   <213> Rhodococcus erythropolis
<400> 16
   Met Asp Gly Ile His Asp Thr Gly Gly Met Thr Gly Tyr Gly Pro Val
   1 5 10 15
   Pro Tyr Gln Lys Asp Glu Pro Phe Phe His Tyr Glu Trp Glu Gly Arg
   20 25 30
   Thr Leu Ser Ile Leu Thr Trp Met His Leu Lys Gly Met Ser Trp Trp
   35 40 45
   Asp Lys Ser Arg Phe Phe Arg Glu Ser Met Gly Asn Glu Asn Tyr Val
   50 55 60
   Asn Glu Ile Arg Asn Ser Tyr Tyr Thr His Trp Leu Ser Ala Ala Glu
   65 70 75 80
   Arg Ile Leu Val Ala Asp Lys Ile Ile Thr Glu Glu Glu Arg Lys His
   85 90 95
   Arg Val Gln Glu Ile Leu Glu Gly Arg Tyr Thr Asp Arg Asn Pro Ser
   100 105 110
   Arg Lys Phe Asp Pro Ala Glu Ile Glu Lys Ala Ile Glu Arg Leu His
   115 120 125
   Glu Pro His Ser Leu Ala Leu Pro Gly Ala Glu Pro Ser Phe Ser Leu
   130 135 140
   Gly Asp Lys Val Lys Val Lys Asn Met Asn Pro Leu Gly His Thr Arg
   145 150 155 160
   Cys Pro Lys Tyr Val Arg Asn Lys Ile Gly Glu Ile Val Thr Ser His
   165 170 175
   Gly Cys Gln Ile Tyr Pro Glu Ser Ser Ser Ala Gly Leu Gly Asp Asp
   180 185 190
   Pro Arg Pro Leu Tyr Thr Val Ala Phe Ser Ala Gln Glu Leu Trp Gly
   195 200 205
   Asp Asp Gly Asn Gly Lys Asp Val Val Cys Val Asp Leu Trp Glu Pro
   210 215 220
   Tyr Leu Ile Ser Ala
   225
<210> 17
   <211> 22
   <212> DNA
   <213> Artificial
<220>
   <223> Primer
<400> 17
   gcccgcataa gaaaaggtga ac 22
<210> 18
   <211> 21
   <212> DNA
   <213> Artificial
<220>
   <223> Primer
<400> 18
   gcatgccttc aaatcagcct g 21
<210> 19
   <211> 24
   <212> DNA
   <213> Artificial
<220>
   <223> Primer
<400> 19
   agggtgaacc atatgtcagt aacg 24
<210> 20
   <211> 22
   <212> DNA
   <213> Artificial
<220>
   <223> Primer
<400> 20
   tgtcggatcc atcagacggt gg 22
<210> 21
   <211> 23
   <212> DNA
   <213> Artificial
<220>
   <223> Primer
<400> 21
   agcaccatat ggatggagta cac 23
<210> 22
   <211> 21
   <212> DNA
   <213> Artificial
<220>
   <223> Primer
<400> 22
   gttgggaatt caggccgcag g 21
<210> 23
   <211> 27
   <212> DNA
   <213> Artificial
<220>
   <223> Primer
<400> 23
   cgcggatcca agaaggagat atacatg 27
<210> 24
   <211> 22
   <212> DNA
   <213> Artificial
<220>
   <223> Primer
<400> 24
   ccgcaacgtt caaacggtct gg 22
<210> 25
   <211> 27
   <212> DNA
   <213> Artificial
<220>
   <223> Primer
<400> 25
   aggaatacgc atatgagcga gcacgtc 27
<210> 26
   <211> 30
   <212> DNA
   <213> Artificial
<220>
   <223> Primer
<400> 26
   gtgtggatcc actcatacga tcacttcctg 30
<210> 27
   <211> 31
   <212> DNA
   <213> Artificial
<220>
   <223> Primer
<400> 27
   aggaatgagc atatggatgg tatccacgac a 31
<210> 28
   <211> 33
   <212> DNA
   <213> Artificial
<220>
   <223> Primer
<400> 28
   atcgggatcc tttcacgcag agatcaggta cgg 33
<210> 29
   <211> 35
   <212> DNA
   <213> Artificial
<220>
   <223> Primer
<400> 29
   ctcaggatcc aaggagtgat cgtatgagtg aagac 35
<210> 30
   <211> 26
   <212> DNA
   <213> Artificial
<220>
   <223> Primer
<400> 30
   acaggagctc tcagtcgatg atggcc 26
<210> 31
   <211> 315
   <212> DNA
   <213> Rhodococcus erythropolis
<220>
   <221> CDS
   <222> (1)..(315)
   <223>
<400> 31
   atg agt gaa gac aca ctc act gat cgg ctc ccg gcg act ggg acc gcc        48
   Met Ser Glu Asp Thr Leu Thr Asp Arg Leu Pro Ala Thr Gly Thr Ala
   1               5                   10                  15
   gca ccg ccc cgc gac aat ggc gag ctt gta ttc acc gag cct tgg gaa        96
   Ala Pro Pro Arg Asp Asn Gly Glu Leu Val Phe Thr Glu Pro Trp Glu
               20                  25                  30
   gca acg gca ttc ggg gtc gcc atc gcg ctt tcg gat cag aag tcg tac       144
   Ala Thr Ala Phe Gly Val Ala Ile Ala Leu Ser Asp Gln Lys Ser Tyr
           35                  40                  45
   gaa tgg gag ttc ttc cga cag cgt ctc att cac tcc atc gct gag gcc       192
   Glu Trp Glu Phe Phe Arg Gln Arg Leu Ile His Ser Ile Ala Glu Ala
       50                  55                  60
   aac ggt tgc gag gca tac tac gag agc tgg aca aag gcg ctc gag gcc       240
   Asn Gly Cys Glu Ala Tyr Tyr Glu Ser Trp Thr Lys Ala Leu Glu Ala
   65                  70                  75                  80
   agc gtg gtc gac tcg ggg ctg atc agc gaa gat gag atc cgc gag cgc       288
   Ser Val Val Asp Ser Gly Leu Ile Ser Glu Asp Glu Ile Arg Glu Arg
                   85                  90                  95
   atg gaa tcg atg gcc atc atc gac tga                                   315
   Met Glu Ser Met Ala Ile Ile Asp
               100
<210> 32
   <211> 104
   <212> PRT
   <213> Rhodococcus erythropolis
<400> 32
   Met Ser Glu Asp Thr Leu Thr Asp Arg Leu Pro Ala Thr Gly Thr Ala
   1               5                   10                  15
   Ala Pro Pro Arg Asp Asn Gly Glu Leu Val Phe Thr Glu Pro Trp Glu
               20                  25                  30
   Ala Thr Ala Phe Gly Val Ala Ile Ala Leu Ser Asp Gln Lys Ser Tyr
           35                  40                  45
   Glu Trp Glu Phe Phe Arg Gln Arg Leu Ile His Ser Ile Ala Glu Ala
       50                  55                  60
   Asn Gly Cys Glu Ala Tyr Tyr Glu Ser Trp Thr Lys Ala Leu Glu Ala
   65                  70                  75                  80
   Ser Val Val Asp Ser Gly Leu Ile Ser Glu Asp Glu Ile Arg Glu Arg
                   85                  90                  95
   Met Glu Ser Met Ala Ile Ile Asp
               100
<210> 33
   <211> 1200
   <212> DNA
   <213> Rhodococcus erythropolis
<220>
   <221> CDS
   <222> (1)..(1200)
   <223>
<400> 33
   atg gtc gac aca cga ctt ccg gtc acg gtg ctg tca ggt ttc ctg ggc      48
   Met Val Asp Thr Arg Leu Pro Val Thr Val Leu Ser Gly Phe Leu Gly
   1               5                   10                  15
   gcc ggg aag acg aca cta ctc aac gag atc ctg cga aat cga gag ggt      96
   Ala Gly Lys Thr Thr Leu Leu Asn Glu Ile Leu Arg Asn Arg Glu Gly
               20                  25                  30
   cgg cgg gtc gcg gtg atc gtc aac gac atg agc gaa atc aac atc gac     144
   Arg Arg Val Ala Val Ile Val Asn Asp Met Ser Glu Ile Asn Ile Asp
           35                  40                  45
   agt gca gaa gtc gag cgt gag atc tcg ctc agt cgc tcc gag gag aaa     192
   Ser Ala Glu Val Glu Arg Glu Ile Ser Leu Ser Arg Ser Glu Glu Lys
       50                  55                  60
   ctg gtc gag atg acc aac ggc tgc atc tgc tgc act ctg cga gag gat     240
   Leu Val Glu Met Thr Asn Gly Cys Ile Cys Cys Thr Leu Arg Glu Asp
   65                  70                  75                  80
   ctt ctt tcc gag atc agc gcc ttg gcc gcc gat ggc cga ttc gac tac     288
   Leu Leu Ser Glu Ile Ser Ala Leu Ala Ala Asp Gly Arg Phe Asp Tyr
                   85                  90                  95
   cta ctc atc gaa tct tcg ggc atc tcc gaa ccg ctt ccc gtc gca gag     336
   Leu Leu Ile Glu Ser Ser Gly Ile Ser Glu Pro Leu Pro Val Ala Glu
               100                 105                 110
   acg ttc aca ttc atc gat acc gac ggc cac gcc ctc gcc gac gtc gcc     384
   Thr Phe Thr Phe Ile Asp Thr Asp Gly His Ala Leu Ala Asp Val Ala
           115                 120                 125
   cga ctc gac acc atg gtc acc gtc gtc gac ggc cac agt ttt ctg cgc     432
   Arg Leu Asp Thr Met Val Thr Val Val Asp Gly His Ser Phe Leu Arg
       130                 135                 140
   gac tac acg gct ggg ggc cgc gtc gaa gcc gat gcc ccg gaa gac gaa     480
   Asp Tyr Thr Ala Gly Gly Arg Val Glu Ala Asp Ala Pro Glu Asp Glu
   145                 150                 155                 160
   cga gac atc gcg gat ctg ctt gtc gat cag atc gaa ttt gcc gac gtc     528
   Arg Asp Ile Ala Asp Leu Leu Val Asp Gln Ile Glu Phe Ala Asp Val
                   165                 170                 175
   atc ctg gtg agc aag gcc gat ctc gtc tcg cac cag cac ctg gtc gaa     576
   Ile Leu Val Ser Lys Ala Asp Leu Val Ser His Gln His Leu Val Glu
               180                 185                 190
   ttg acc gca gtc ctg cgc tct ttg aac gca tcc gct gcg ata gtt ccg     624
   Leu Thr Ala Val Leu Arg Ser Leu Asn Ala Ser Ala Ala Ile Val Pro
           195                 200                 205
   atg acg ctc ggt cgc atc cca ctc gac acg att ctc gac acc ggt ttg     672
   Met Thr Leu Gly Arg Ile Pro Leu Asp Thr Ile Leu Asp Thr Gly Leu
       210                 215                 220
   ttc tcg ctc gaa aag gct gca cag gcc ccc gga tgg tta caa gaa ctc     720
   Phe Ser Leu Glu Lys Ala Ala Gln Ala Pro Gly Trp Leu Gln Glu Leu
   225                 230                 235                 240
   caa ggt gaa cac atc ccc gaa acc gaa gag tac gga atc agt tcg gtg     768
   Gln Gly Glu His Ile Pro Glu Thr Glu Glu Tyr Gly Ile Ser Ser Val
                   245                     250                     255
   gtg tac cgc gag cgc gca ccc ttc cac ccc caa cgg ctg cat gat ttc        816
   Val Tyr Arg Glu Arg Ala Pro Phe His Pro Gln Arg Leu His Asp Phe
               260                 265                 270
   ctc agc agc gag tgg acc aac gga aag tta ctt cgg gcc aag ggc tac        864
   Leu Ser Ser Glu Trp Thr Asn Gly Lys Leu Leu Arg Ala Lys Gly Tyr
           275                 280                 285
   tac tgg aat gcc ggc cgg ttc acc gag atc ggg agt att tct cag gcc        912
   Tyr Trp Asn Ala Gly Arg Phe Thr Glu Ile Gly Ser Ile Ser Gln Ala
       290                 295                 300
   ggt cat ctc att cgc cac gga tac gtc ggc cgt tgg tgg aag ttt cta        960
   Gly His Leu Ile Arg His Gly Tyr Val Gly Arg Trp Trp Lys Phe Leu
   305                 310                 315                 320
   ccc cgt gac gag tgg ccg gcc gac gat tac cgt cgt gac gga atc ctc       1008
   Pro Arg Asp Glu Trp Pro Ala Asp Asp Tyr Arg Arg Asp Gly Ile Leu
                   325                 330                 335
   gac aag tgg gaa gaa ccc gtc gga gac tgc cga caa gaa ctc gtc ttc       1056
   Asp Lys Trp Glu Glu Pro Val Gly Asp Cys Arg Gln Glu Leu Val Phe
               340                 345                 350
   atc ggc caa gcc atc gac ccg tct cga ctg cac cga gaa ctc gac gcg       1104
   Ile Gly Gln Ala Ile Asp Pro Ser Arg Leu His Arg Glu Leu Asp Ala
           355                 360                 365
   tgt cta ctc acc aca gcc gag atc gaa ctc ggg cca gac gtg tgg acc       1152
   Cys Leu Leu Thr Thr Ala Glu Ile Glu Leu Gly Pro Asp Val Trp Thr
       370                 375                 380
   acc tgg agc gac ccc ctg ggc gtc ggc tat acc gac cag acc gtt tga       1200
   Thr Trp Ser Asp Pro Leu Gly Val Gly Tyr Thr Asp Gln Thr Val
   385                 390                 395
<210> 34
   <211> 399
   <212> PRT
   <213> Rhodococcus erythropolis
<400> 34
   Met Val Asp Thr Arg Leu Pro Val Thr Val Leu Ser Gly Phe Leu Gly
   1 5 10 15
   Ala Gly Lys Thr Thr Leu Leu Asn Glu Ile Leu Arg Asn Arg Glu Gly
   20 25 30
   Arg Arg Val Ala Val Ile Val Asn Asp Met Ser Glu Ile Asn Ile Asp
   35 40 45
   Ser Ala Glu Val Glu Arg Glu Ile Ser Leu Ser Arg Ser Glu Glu Lys
   50 55 60
   Leu Val Glu Met Thr Asn Gly Cys Ile Cys Cys Thr Leu Arg Glu Asp
   65 70 75 80
   Leu Leu Ser Glu Ile Ser Ala Leu Ala Ala Asp Gly Arg Phe Asp Tyr
   85 90 95
   Leu Leu Ile Glu Ser Ser Gly Ile Ser Glu Pro Leu Pro Val Ala Glu
   100 105 110
   Thr Phe Thr Phe Ile Asp Thr Asp Gly His Ala Leu Ala Asp Val Ala
   115 120 125
   Arg Leu Asp Thr Met Val Thr Val Val Asp Gly His Ser Phe Leu Arg
   130 135 140
   Asp Tyr Thr Ala Gly Gly Arg Val Glu Ala Asp Ala Pro Glu Asp Glu
   145 150 155 160
   Arg Asp Ile Ala Asp Leu Leu Val Asp Gln Ile Glu Phe Ala Asp Val
   165 170 175
   Ile Leu Val Ser Lys Ala Asp Leu Val Ser His Gln His Leu Val Glu
   180 185 190
   Leu Thr Ala Val Leu Arg Ser Leu Asn Ala Ser Ala Ala Ile Val Pro
   195 200 205
   Met Thr Leu Gly Arg Ile Pro Leu Asp Thr Ile Leu Asp Thr Gly Leu
   210 215 220
   Phe Ser Leu Glu Lys Ala Ala Gln Ala Pro Gly Trp Leu Gln Glu Leu
   225 230 235 240
   Gln Gly Glu His Ile Pro Glu Thr Glu Glu Tyr Gly Ile Ser Ser Val
   245 250 255
   Val Tyr Arg Glu Arg Ala Pro Phe His Pro Gln Arg Leu His Asp Phe
   260 265 270
   Leu Ser Ser Glu Trp Thr Asn Gly Lys Leu Leu Arg Ala Lys Gly Tyr
   275 280 285
   Tyr Trp Asn Ala Gly Arg Phe Thr Glu Ile Gly Ser Ile Ser Gln Ala
       290                 295                 300
   Gly His Leu Ile Arg His Gly Tyr Val Gly Arg Trp Trp Lys Phe Leu
   305                 310                 315                 320
   Pro Arg Asp Glu Trp Pro Ala Asp Asp Tyr Arg Arg Asp Gly Ile Leu
                   325                 330                 335
   Asp Lys Trp Glu Glu Pro Val Gly Asp Cys Arg Gln Glu Leu Val Phe
               340                 345                 350
   Ile Gly Gln Ala Ile Asp Pro Ser Arg Leu His Arg Glu Leu Asp Ala
           355                 360                 365
   Cys Leu Leu Thr Thr Ala Glu Ile Glu Leu Gly Pro Asp Val Trp Thr
       370                 375                 380
   Thr Trp Ser Asp Pro Leu Gly Val Gly Tyr Thr Asp Gln Thr Val
   385                 390                 395